# EUROPEAN PATENT APPLICATION

(11) **EP 2 468 735 A1**
(43) Date of publication of application: **27.06.2012**
(21) Application number: 10016009.2
(22) Date of filing: 23.12.2010
(51) Int. Cl.: C07D 263/24, C07C 43/205, C07C 41/18, C07C 41/20, C07C 41/22, A61K 31/4166

(54) **Synthesis of intermediates for preparing anacetrapib and derivates thereof**

(71) Applicant: LEK Pharmaceuticals d.d., 1526 Ljubljana (SI)
(72) Inventor: The designation of the inventor has not yet been filed
(74) Representative: Hodzar, Damjan

(57) **Abstract**

The present invention relates to the field of organic chemistry, more specifically to the synthesis of intermediate compounds which can be used in the synthesis of pharmaceutically active agents such as anacetrapib or derivatives thereof.

## Description

### Field of the invention

The present invention relates to the field of organic chemistry, more specifically to the synthesis of intermediate compounds which can be used in the synthesis of pharmaceutically active agents such as anacetrapib or derivatives thereof.

### Background of the invention

Anacetrapib (chemically named (4S,5R)-5-[3,5-bis(trifluoromethyl)phenyl]-3-[[2-(4-fluoro-2-methoxy-5-propan-2-ylphenyl)-5-(trifluoromethyl)phenyl]methyl]-4-methyl-1,3-oxazolidln-2-one) having the structural formula 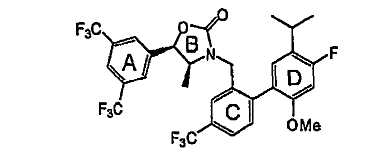 has been shown to act as an inhibitor of cholesteryl ester transfer protein (CETP). The complex molecular structure of anacetrapib comprises four cycles A, B, C and D as shown above, wherein this structure can be built up in various manners. The following linear (A + B + C + D) and convergent approach (AB + CD) for the synthesis of anacetrapib have been disclosed in patent applications WO 20061014413 and WO 2007/005572, respectively. In the preferred embodiments of both aforementioned approaches, the compounds shown in Scheme 1 wherefrom cycle moieties C and D derive, are used as the starting material 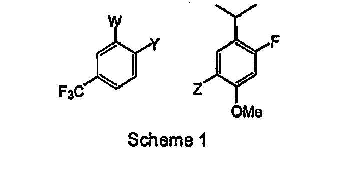 , wherein substituents Y and Z represent groups suitable for biaryl coupling, and W is a group which is CH₂Q or a group which can be converted to CH₂Q, In which Q is halo, hydroxy, substituted hydroxy, amino or substituted amino for the direct coupling to another molecule such as a mullicycle molecule comprising cycles A and B.

In WO 2006/014413 and WO 2007/005572, 1-halo-4-fluoro-5-isopropyl-2-methoxybenzene as shown in Scheme 2 (**XMIP**, wherein X is bromo or iodo) Is used as the common key intermediate for coupling of fragment D.

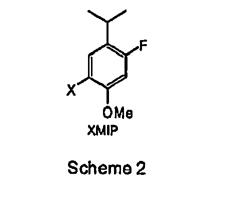

The convergent synthesis described in WO 2007/005572 in which biaryl derivative is prepared from 1-(2-fluoro-4-methoxyphenyl)ethanone (**FMAP**) requires a three-step process to obtain 1-bromo-4-fluoro-5-isopropyl-2-methoxybenzene (**BrMIP**) as show in Scheme 3 and appears to be the most favorable approach.

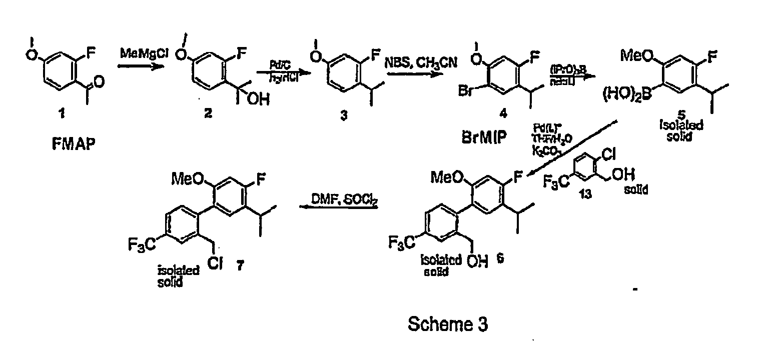

However, the methods described in the aforementioned patent applications have some drawbacks regarding impurity profile, yields and the use of genotoxic solvents. It has been surprisingly found that the method described in WO 2007/005572 leads to an impure final product, which is hard to purify to a pharmaceutical grade by conventional purification methods. Our careful analysis discovered an impurity desmethylanacetrapib (**DMAP**), a compound having the structural formula 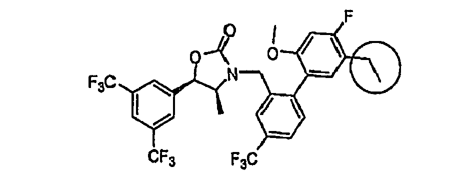 , wherein **DMAP** differs from anacetrapib in that cycle D is substituted with an ethyl moiety (indicated with a circle) instead of an isopropyl moiety.

As shown in Scheme 4, desmethylanacetraplb (**DMAP**) originates from a very early step of the synthesis described in WO 2007/005572. 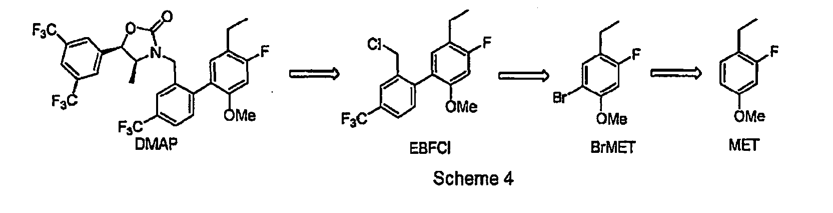 , wherein 1-ethyl-2-fluoro-4-methoxybenzene (**MET**) is a result of incompleteness of Gdgnard methylation of acetophenone from which the remaining starting material 1-(2-fluoro-4-methoxyphenyl)ethanone (**FMAP**) is converted to **MET** (Scheme 5). Furthermore, when reproducing the prior art process it was observed that approximately 5-10 % of **FMAP** remains in the reaction process and that the impurity **MET** formed can not be removed in the following steps. Moreover, **MET** is first transformed to 1-bromo-5-ethyl-4-ftuoro-2-methoxybenzene (**BrMET**) and then to chloro biaryl impurity **EBFCI,** which after coupling with heterocyclic intermediate results in the additional impurity desmethylanacetrapib (**DMAP**) in the final product anacetrapib.

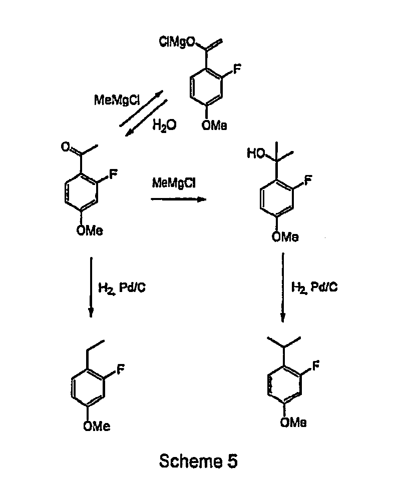

In order to overcome the above described problem, it was suggested to use anhydrous cerium (III) chloride, which is highly hydroscopic and thus not feasible and/or economical for the usage in industrial scale. Furthermore, while repeating this process, the reaction is too slow and not complete.

Another synthesis described in WO 2007/136672 starts from 1-bromo-2,4-difluorobenzene and requires a four-step process to obtain 1-bromo-4-fluoro-5-isopropyl-2-methoxybenzene (**BrMIP**) as shown in Scheme 6.

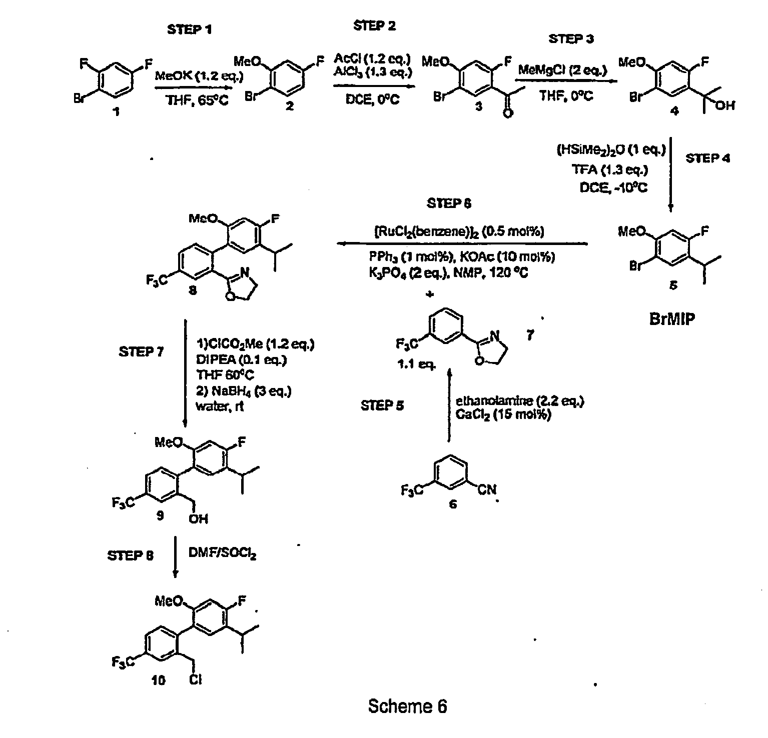

The critical step of grignardation is performed on the corresponding bromo derivative, what can have some advantages in view of desmethyl impurities. However, the propanol derivative 4 cannot be simply hydrogenated to isopropyl derivative due to considerable debromination (Scheme 7).

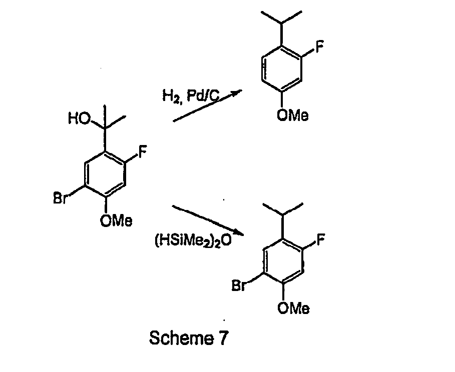

Consequently, said conversion should be performed using tetramethyldisiloxane (TMDSO), which is less suitable reagent for industrial use. Additionaliy, applying TMDSO leads to modest yields and the formation of siloxane impurities that are difficult to remove. Further drawbacks of the whole synthetic scheme of WO 2007/136672 are the use of dichloroethane as a solvent, since dichloroethane is reasonably anticipated to be human carcinogen, and the use of uneconomical ruthenium catalysts.

Therefore, there is an unmet need for a preparation of highly pure intermediates for the synthesis of cholesterylester transfer protein (CETP) inhibitors such as anacetrapib.

### Summary of the invention

Various aspects, advantageous features and preferred embodiments of the present Invention as summarized in the following items, respectively alone and in combination, contribute to solving the object of the invention.
(1) A process for preparing a compound of formula II 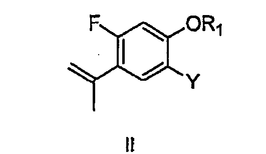 , wherein R₁ is substituted or unsubstituted C₁-C₆ alkyl and Y is Cl, Br or I**,**
   by dehydration of a compound of formula I 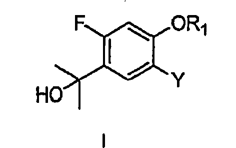 , wherein R₁ and Y are defined as above,
   in the presence of a proton donator.
   The term "alkyl" as used herein means straight or branched chain alkyl moiety.
   The term "substituted" as used herein means that one or more, preferably up to 5, more preferably 1-3 hydrogen atoms of a structural moiety are replaced independently from each other by the corresponding number of substituents. Typical substituents include, without being limited thereto, for example halogen, trifluoromethyl, cyano, nitro, oxo, NR', -OR', -C(O)R', -C(O)OR', -OC(O)R', -S(O)R', N(R')R", C(O)N(R')R", - SO₂N(R')R' and R''', wherein each of R', R" and R''' are selected from the group consisting of C₁ - C₆ alkyl, C₁ - C₆ alkoxy, -(CH₂)ₘ-heterocyclyl (m being 1, 2, 4 or 4) and each R' and R" may be optionally and independently further substituted with one or more of hydrogen, halogen, cyano, amino, hydroxy, C₁ - C₆ alkyl and C₁ - C₆ alkoxy. Specific substituents in particular include halogen such as fluoro, chloro and/or bromo, hydroxy, amino, C₁ - C₆ alkyl and C₁ - C₆ alkoxy, and halogenated C₁ - C₆ alkyl and C₁ - C₆ alkoxy such as trifiuoro-methyl. It will be understood that the substituents are at positions where their introduction is chemically possible, that is positions being known or evident to the person skilled in the art to decide (either experimentally or theoretically) without inappropriate effort whether a particular substitution is possible. For example, substituents which may be unstable or may affect reactions disclosed herein may be omitted at least at a critical reaction step.
   The term "dehydration" as used herein means an elimination reaction involving the loss or elimination of water from a starting compound, wherein an alkene compound is formed as the reaction product.
   The term "proton donator" as used herein means a compound capable of donating a proton, that is a Bronsted acid.
   The procedural concept according to this aspect of the invention provides for a compound of formula 11 representing a highly valuable intermediate for the preparation of pharmaceutically active agents such as anacetrapib or derivatives thereof, since compound of formula II has a high purity owing to the simple and efficient dehydration reaction wherein no difficultly removable side products are formed. Furthermore, compound of formula II represents a versatile intermediate compound, since its isopropenyl group may be easily converted to isopropyl group before or after reacting compound of formula II with another compound.
(2) The process according to item (1), wherein In compound of formula I and II R₁ is C₁-C₃ alkyl and Y is Cl, Br or I, preferably R₁ is methyl (Me) and Y is Br.
(3) The process according to item (1) or (2), wherein dehydration is carried out in the presence of water, preferably the water is provided by an aqueous solution of the proton donator, more preferably the proton donator is in form of an aqueous solution comprising 0.01 to 10 mol proton donator per liter of aqueous solution of proton donator, even more preferably 0.1 to 5 mol proton donator per liter, and in particular 0.6 to 3 mol proton donator per liter.
(4) The process according to any one of the preceding items, wherein water and/or compound of formula II is removed from the reaction mixture during dehydration reaction, preferably water and compound of formula II is removed from the reaction mixture during dehydration reaction.
   According to this embodiment of the invention, the chemical equilibrium of the dehydration reaction is forced to the product side, providing for both time-efficient preparation and quantitative or at least substantially quantitative conversion of compound of formula I to compound of formula II.
(5) The process according to item (4), wherein removal is carried out by distillation, preferably steam distillation of water and product.
   The term "steam distillation" as used herein means that a mixture of two (or more) fluid components, in this case at least two components selected from water, reaction product in form of compound of formula II and optionally solvent applied in the dehydration reaction, is separated from the reaction mixture by distillation. Preferably, water and compound of formula II are distilled off from the reaction mixture, wherein an optionally applied solvent In the dehydration reaction and starting material in form of compound of formula I remain in the reaction vessel. Removal of the fluid compounds can be achieved by suitable distillation apparatus, for example by Dean-Stark distillation apparatus.
   This measure provides for an efficient and economical isolation of the reaction product in the case when compound of formula II alone or in combination with water and/or solvent is removed from the reaction mixture by distillation, since no elaborate purification steps such as chromatography and/or recrystallisation are required.
(6) The process according to any one of the preceding items, wherein the reaction mixture is heated to the boiling point of mixture of water and reaction product.
(7) The process according to any one of the preceding items, wherein the proton donator is selected from organic or inorganic acids, preferably inorganic acids, more preferably inorganic acids selected from the group consisting of HCl, H₂SO₄, H₃PO₄ and NaHSO₄, in particular the proton donator is H₂SO₄.
(8) The process according to any one of the preceding items, wherein the dehydration reaction is optionally carried out in the presence of an organic solvent, preferably an organic solvent, miscible with water and inert to acidic conditions which boiling point is at least 60°C higher than the boiling point of the mixture of water and of compound of formula II, more preferably an organic solvent which boiling point is at least 100°C higher than the boiling point of the mixture of water and of compound of formula II, even more preferably the organic solvent is non-volatile under applied dehydration reaction conditions.
(9) The process according to item (8), wherein the organic solvent is ethylene glycol, polyethylene glycol (PEG), sulfolane, DMSO, preferably a polyethylene glycol having an average molecular weight of 100 to 8000, more preferably a polyethylene glycol having an average molecular weight of 300 to 3000, and in particular a polyethylene glycol having an average molecular weight of about 400.
(10) A process for preparing a compound of formula III, wherein compound of formula II 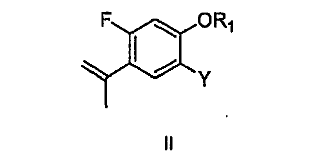 wherein R₁ is substituted or unsubstituted C₁-C₆ alkyl and Y is Cl, Br or I,
   is converted to compound of formula III 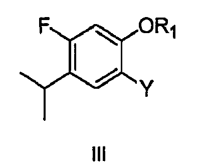 wherein R₁ and Y are defined as above,
   by hydrogenation in the presence of a hydrogenation catalyst.
   The term "hydrogenation" as used herein means a chemical reaction wherein a starting compound is reduced by contacting with hydrogen.
   According to this preferred aspect of the invention, a compound of formula III is provided which represents a highly valuable intermediate for the preparation of pharmaceutically active agents such as anacetrapib or derivatives thereof, since compound of formula III is attained in both high yields and exceptional purity. In particular, compound of formula III can be obtained free of siloxane impurities and has a content of desmethyl impurity in form of 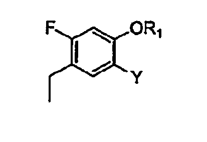 , wherein R₁ and Y are defined as above,
   of less than 0.50% by weight, preferably less than 025 %, and more preferably less than 0.15 %.
   Compound II is a particularly suitable starting material for preparing compound of formula III, since the above mentioned hydrogenation of compound of formula li provides for faster reaction rates while requiring a lower load of hydrogenation catalyst compared to a reduction reaction of compound of formula I to compound of formula III in the presence of hydrogen and catalyst.
(11) The process according to item (10), wherein hydrogenation is carried out in organic solvents, preferably in a C₁-C₆ alcohol as the solvent
(12) The process according to item (10) or (11), wherein hydrogenation is carried out using palladium as the hydrogenation catalyst, preferably palladium supported on a solid, more preferably palladium on activated charcoal.
(13) The process according to any one of items (10) to (12), wherein HBr or a catalyst poison is present in the solvent, more preferably the catalyst poison is a sulfur compound, even more preferably the catalyst poison is an organic sulfur component, and in particular the organic sulfur component is thiophen.
   According to this embodiment of the invention, exceptionally pure compound of formula III is obtained. This is because dehalogenation of compounds of formulae II and III is effectively inhibited, and thus, it is substantially free of dehalogenated byproducts which are difficult to remove.
(14) The process according to any one of items (10) to (13), wherein said conversion follows subsequent to a process as defined in any one of items (1) to (9).
(15) The process according to any of the items (1) to (9), wherein compound of fonnula II 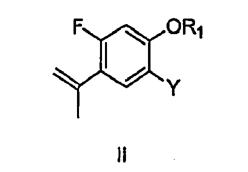 , wherein R₁ substituted or unsubstituted is C₁-C₆ alkyl and Y is Cl, Br or I, is converted to compound of formula V 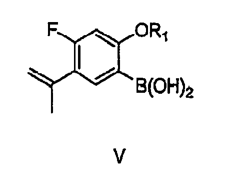 , wherein R₁ is defined as above,
   by treating compound of formula II with trialkyl borate in the presence of an organolithium or lithium in an inert aprotic solvent or mixture of solvents, preferably the trialkyl borate is triisopropyl borate.
(16) The process according to any one of items (10) to (14), wherein the compound of formula III 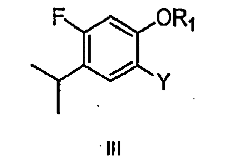 , wherein R₁ is substituted or unsubstituted C₁-C₆ alkyl and Y is Cl, Br or I, is converted to compound of formula IV 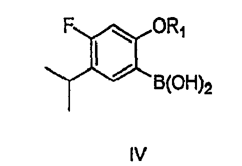 ,wherein R₁ is defined as above,
   by treating compound of formula III with trialkyl borate in the presence of an organolithium or lithium in an inert aprotic solvent or mixture of solvents, preferably the trialkyl borate is triisopropyl borate.
   According to a preferred embodiment, the used compound of formula IV is free of siloxanes and has a content of desmethyl impurity in form of 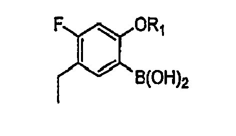 , wherein R₁ Is defined as above,
   of less than 0.50% by weight, preferably less than 0.25 %, and more preferably less than 0.15 %.
(17) The process according to item (15) or (16), wherein organolithium compound is BuLi.
(18) The process according to item (15) or (16), wherein lithium in C₅-C₇-alkane is used.
(19) The process according to any one of items (15) to (18), wherein treatment is carried out at a temperature below -25 °C, preferably below -50 °C.
(20) The process according to any one of items (16) to (19), wherein the compound of formula IV 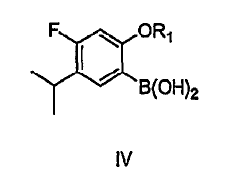 wherein R₁ is substituted or unsubstituted C₁-C₆ alkyl, is coupled with a compound of formula VI, 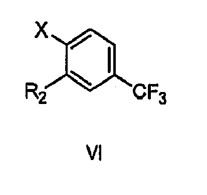 wherein X is Cl, Br, I, -O-SO₂-CF₃(-OTf), -N₂⁺ or -N(Me)₃⁺, R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, hatogenmethyl, carboxy, alkoxycarbonyl, amidocarbonyl, cyano, formyl, 2-(4,5-dihydro-1,3-oxazolyl) and nitro,
   to form a compound of formula VII₁ 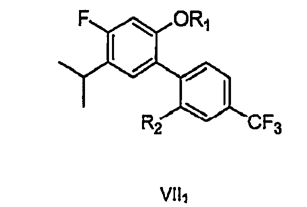 wherein R₁ and R₂ are defined as above.
   The terms "leaving group" and "a group convertible to a leaving group" used herein mean a group which is easily substituted by nucleophile substitution reaction. A "group convertible to a leaving group" has the capability of being conventionally converted to a desired leaving group. Optionally the respective leaving group of a precursor group thereof is protected by usual and known protection groups. The respective meanings of these terms become further apparent from the more specific definitions provided herein In the disclosure of preferred embodiments.
   The term "halogen" as used herein means the group consisting of chloro, bromo and iodo.
   The term "acyl or sulfonyl substituted hydroxy" as used herein means a carboxylic acid ester -O-C(O)-R₄ and a sulfonic acid ester -O-SO₂-R₄ moiety respectively, wherein R₄ is substituted or unsubstituted C₁-C₂₀ alkyl or C₅-C₂₀ aryl, preferably substituted or unsubstituted C₁-C₁₀ alkyl or C₆-C₁₂ aryl.
   The term "acyl or sulfonyl substituted amino" as used herein means an amide -N(R₅)-CO-R₆ and a sulfonamide -N(R₅)-SO₂-R₆ group respectively, wherein R₅ is H, substituted or unsubstituted C₁-C₆ alkyl or substituted or unsubstituted C₅-C₁₂ aryl, preferably H, substituted or unsubstituted C₁-C₃ alkyl or substituted or unsubstituted C₆-C₁₀ aryl, and R₆ is selected from the group consisting of substituted or unsubstituted C₁-C₂₀ alkyl, substituted or unsubstituted C₅-C₂₀ aryl and camphoryl, preferably substituted or unsubstituted C₁-C₁₀ alkyl, substituted or unsubstituted C₆-C₁₂ aryl and camphoryl.
   The term "alkoxy" as used herein means -O-alkyl, wherein substituted or unsubstituted alkyl is straight or branched chain and comprises 1 to 6 carbon atoms, preferably 1 to 4 carbon atoms.
   The term "aryl" as used herein means substituted or unsubstituted aromatic ring systems comprising 6 to 16 carbon atoms located within the aromatic ring system. Preferably, the substituted or unsubstituted aromatic ring system comprises 1 to 3 aromatic rings, more preferably, the aromatic ring system is selected from the group consisting of phenyl, naphthyl, fluorenyl, azulenyl, indenyl, anthryl, and in particular, the aromatic ring system is phenyl.
   The term "substituted" as used herein in connection with the above definitions of the terms "acyl or sulfonyl substituted hydroxyl","acyl or sulfonyl substituted amino" and "alkoxy" has the same meaning as the term "substituted" explained under item (1) for R₁ group.
(21) The process according to any one of Items (15) and (17) to (19), wherein compound of formula V 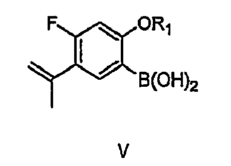 , wherein R₁ is substituted or unsubstituted C₁-C₆ alkyl,
   is coupled with a compound of formula VI 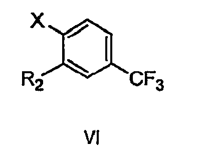 wherein X is Cl, Br, I, -O-SO₂-CF₃ (-OTf), -N₂⁺ or -N(Me)₃⁺, wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymefhyl, alkoxymethyl, halogenmethyl, carboxy, alkoxycarbonyl, amidocarbonyl, cyano, formyl, 2-(4,5-dihydro-1,3-oxazolyl) and nitro,
   to form a compound of formula VII₂, 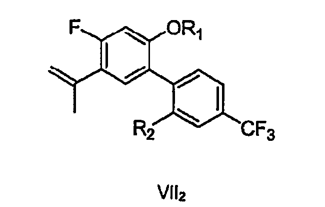 , wherein R₁ and R₂ are defined as above,
   in the presence of a coupling catalyst.
   As to the meaning of the terms "weaving group", "halogen", "acyl or sulfonyl substituted hydroxy", "acyl or sulfonyl substituted amino" and "alkoxy" as used herein, reference is made to the explanations under item (20) above.
(22) The process according to any one of items (10) to (14), wherein the compound of formula III 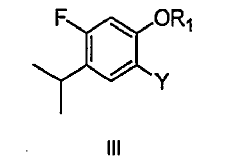 , wherein R₁ is substituted or unsubstituted C₁-C₆ alkyl and Y is Cl, Br or I,
   is converted to compound of formula VII₁ 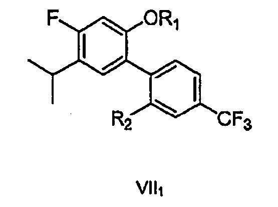 wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,S-dihydro-1,3-oxazolyl) and nitro,
   in an one-pot reaction
   by treating compound of formula III with trialkyl borate in the presence of an organolithium or lithium in an inert aprotic solvent or mixture of solvents followed by an addition of compound of formula VI 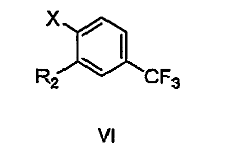 wherein X is Cl, Br, I, -O-SO₂-CF₃ (-OTf), -N₂⁺ or -N(Me)₃⁺, wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   in the presence of a coupling catalyst.
   As to the meaning of the terms "leaving group", "halogen", "acyl or sulfonyl substituted hydroxy", "acyl or sulfonyl substituted amino" and "alkoxy" as used herein, reference is made to the explanations under item (20) above.
   The term "one-pot reaction" as used herein means that at least two reactions are subsequently carried out without isolating intermediate compounds.
(23) The process according to any of the items (1) to (9), wherein the compound of formula II 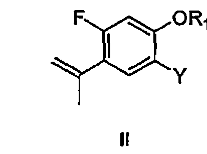 , wherein R₁ is substituted or unsubstituted C₁-C₆ alkyl and Y is Cl, Br or I,
   is converted to compound of formula VII₂ 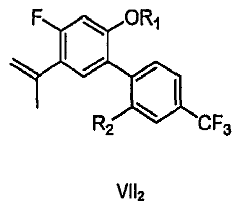 wherein R₁ is substituted or unsubstituted C₁-C₆ alkyl and R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   in an one-pot reaction
   by treating compound of formula II with trialkyl borate In the presence of an organolithium or lithium in an inert aprotic solvent or mixture of solvents followed by an addition of compound of formula VI 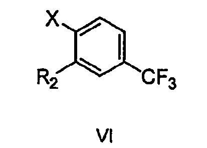 wherein X is Cl, Br, I, -O-SO₂-CF₃ (-OTf), -Nz⁺ or -N(Me)₃⁺, wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amldocarbonyl, cyano, formyl, 2-(4,5-dihydro-1,3-oxazolyi) and nitro,
   in the presence of a coupling catalyst.
   As to the meaning of the terms "leaving group", "halogen", "acyl or sulfonyl substituted hydroxy", "acyl or sulfonyl substituted amino" and "alkoxy" as used herein, reference is made to the explanations under item (20) above.
   As to the meaning of the term "one-pot reaction", reference is made to the explanations under item (22) above.
   According to the beneficial and preferred embodiments defined in items (22) and (23) above, particularly efficient processes for preparing biaryl compounds are provided, since these processes surprisingly enable omission of isolation of boronic acid compounds of formula IV and V as defined in the previous items, while highly pure products are obtained. Surprisingly, side products originating from the two subsequently carried out reaction steps, such as alkyhalogenide or alkylboronic acid in the first reaction step wherein the boronic acid intermediate compound is formed, and side products formed during the first step do not substantially interfere with the second step of Suzufci coupling, and all the impurities formed In the one pot reaction can be fully removed by one final purification step, preferably crystallization without further efforts.
(24) The process according to item (22) or (23), wherein trialkyl borate is used in an amount of 1.0 to 2.0 equivalents relative to compound of formula II or III and organolithium compound is used in a amount of 1.0 to 1.5 equivalents relative to compound of formula II or III.
(25) The process according to any one of items (22) to (24), wherein trialkyl borate is triisopropyl borate.
(26) The process according to any one of items (22) to (25), wherein the organolithium compound is n-butylithlum.
(27) The process according to any one of items (22) to (25), wherein instead of organolithium compound, lithium in C₅-C₇-alkane is used.
(28) The process according to any one of items (22) to (27), wherein the resulting reaction mixture is maintained at a temperature below -25 °C, preferably below -50 °C.
(29) The process according to any one of items (22) to (28), wherein the coupling catalyst is selected from the group consisting of palladium-triaryiphosphine, -trialkylphosphine or -aryl and alklyl substituted phosphine complexes with palladium in the zero oxidation state, salts of palladium in the presence of phosphine ligands, and metallic palladium optionally supported on a solid, preferably the catalyst Is Pd(OAc)₂ / PPh₃ or 1,1, bis(di-tertbutylphosphino)ferrocene palladium dichloride.
   The term "alkyl" as used herein means a straight or branched chain alkyl moiety having 1 to 6 carbon atoms, preferably 1 to 4 carbon atoms.
   The term "aryl" as used herein means substituted or unsubstituted aromatic ring systems comprising 5 to 16 carbon atoms located within the aromatic ring system. Preferably, the substituted or unsubstituted aromatic ring system comprises 1 to 3 aromatic rings, more preferably, the aromatic ring system is selected from the group consisting of phenyl, ferrocenyl, naphthyl, fluorenyl, azulenyl, indenyl, anthryl, and in particular, the aromatic ring system is phenyl.
(30) The process according to item (23), wherein the compound of formula VII₂ 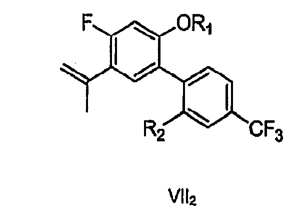 , wherein R₁ is substituted or unsubstituted C₁-C₆ alkyl and R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   is converted to compound of formula VII₁, 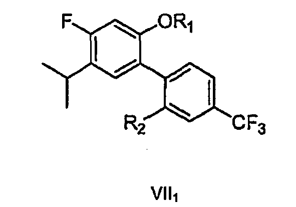 ,wherein R₁ and R₂ are defined as above,
   by hydrogenation of compound of formula VII₂ in the presence of a hydrogenation catalyst.
   As to the meaning of the term "hydrogenation", reference is made to item (10) above.
   According to a preferred embodiment, the used compound of formula IV is free of siloxane and has a content of desmethyl impurity in form of 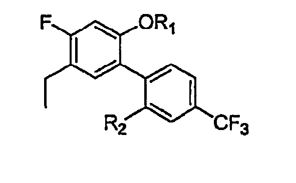 , wherein R₁ and R₂ are defined as above,
   of less than 0.50% by weight preferably less than 0.25 %, and more preferably less than 0.15 %.
(31) The process according to item (23), wherein the compound of formula VII₂' 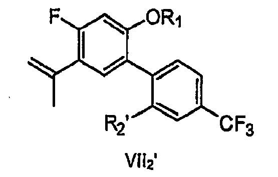 wherein R₁ is substituted or unsubstituted C₁-C₆ alkyl and R₂' is a group which can be reduced, preferably hydrogenated simultaneously with the 2-propenyl group of formula VII₂' in order to form a group R₂" representing -CH₂Q wherein Q is a leaving group, preferably R₂ Is selected from the group consisting of formyl, cyano, alkoxycarbonyl and amidocarbonyl,
   is converted to compound of formula VII₁' 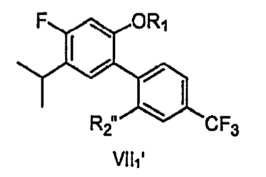 wherein R₁ is defined as above, and R₂" represents the reduced, preferably hydrogenated form of R₂'. preferably -CH₂OH or -CH₂NH₂,
   by reduction, preferably hydrogenadon of compound of formula VII₂ in the presence of reducing agent, preferably hydrogen and a hydrogenation catalyst
   As to the meaning of the terms "leaving group", reference is made to the explanations under item (20) above.
   As to the meaning of the term "one-pot reaction", reference is made to the explanations under item (22) above.
   The term "reduction" as used herein means a chemical reaction wherein a starting compound is chemically reduced.
   As to the meaning of the term "hydrogenation", reference is made to the explanations under item (10) above.
   In this way, a particularly efficient process for preparing biaryl compounds representing building blocks for more complex target molecules is provided, since the special selection of group R₂ in form of R₂' provides for simultaneous hydrogenation of both isopropenyl group and R₂' group of compound of formula VII₂', wherein a reactive group R₂" providing a leaving group Q for reactivity to other compounds in subsequent steps is introduced, while only one hydrogenation/reduction step is required, and thus also one work-up and purification step is saved.
(32) The process according to any one of items (15) and (17) to (19), wherein the compound of formula V 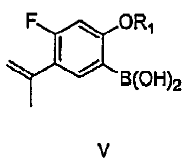 , wherein R₁ is substituted or unsubstituted C₁-C₆ alkyl,
   is converted to a compound of formula IV 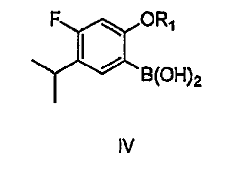 , wneretn R₁ is defined as above,
   by hydrogenating compound of formula V in the presence of a hydrogenation catalyst.
   According to a preferred embodiment, the used compound of formula IV is free of siloxane and has a content of desmethyl impurity in form of 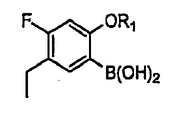 wherein R₁ is defined as above, of less than 0.50% by weight, preferably less than 0.25 %, and more preferably less than 0.15 %.
(33) The process according to any one of items (30) to (32), wherein the hydrogenation catalyst is Raney nickel, preferably activated Raney nickel.
(34) The process according to item (33), wherein catalysis is carried out in NH₄OH as the solvent.
(35) The process according to any one of items (30) to (32), wherein the hydrogenation catalyst is a palladium catalyst.
(36) The process according to item (35), wherein the palladium catalyst is supported on a solid, more preferably the catalyst is palladium on activated charcoal.
(37) The process according to item (35) or (36), wherein hydrogenation is carried out in organic solvents, preferably C₁-C₆ alcohol as the solvent, more preferably C₁-C₃ alcohol, in particular methanol.
(38) A compound of formula II 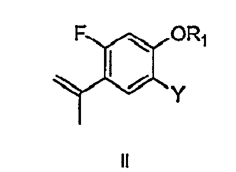 , wherein R₁, is substituted or unsubstituted C₁-C₆ alkyl and Y is Cl, Br or I.
(39) A compound of formula V 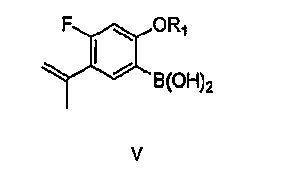 , wherein R₁, is substituted or unsubstituted C₁-C₆ alkyl, and salts and esters thereof.
(40) A compound of formula VII₂, 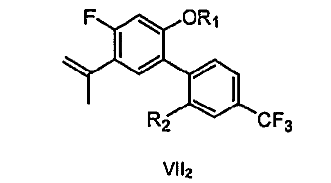 , wherein R₁ is substituted or unsubstituted C₁-C₆ alkyl, and R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymothyl, helogenmethyl, carboxy, alkoxycarbonyl, amidocarbonyl, cyano, formyl, 2-(4,5-dihydro-1,3-oxazolyl) and nitro, even more preferably R₂ is cyano, hydroxymethyl, formyl, nitro, and aminomethyl.
   As to the meaning of the terms "leaving group", "halogen", "acyl or sulfonyl substituted hydroxy", "acyl or sulfonyl substituted amino" and "alkoxy" as used herein, reference is made to the explanations under item (20) above.
(41) The compound according to any one of items (38) to (40), wherein the content of desmethyl impurity in the compound is less than 0.50% by weight, preferably less than 0.25%, and more preferably less than 0.15%.
   The term "desmethyl impurity" as used herein means compound of formula II, IV or VII comprising an ethyl or acetyl group instead of the isopropyl or isopropenyl group. Such impurities originate from unreacted starting material e.g. of a Grignard reaction as elucidated in Schemes 4 and 5 above.
(42) A compound of formula VII₁, 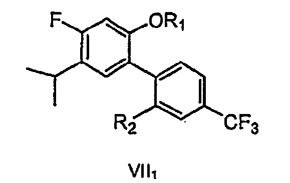 , wherein R₁ is substituted or unsubstituted C₁-C₆ alkyl, and R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro, even more preferably R₂ is cyano, hydroxymethyl, formyl, nitro, aminomethyl,
   wherein compound of formula VII₁ is free of siloxane impurity and a content of desmethyl impurity in the compound is less than 0.50% by weight, preferably less than 0.25 % by weight, and more preferably less than 0.15 % by weight.
   The term "siloxane impurity" as used herein means siloxane byproducts forming in a reduction reaction carried out with an alkylsiloxane, e.g a reduction as elucidated In Scheme 7 above.
   As to the meaning of the terms "leaving group", "halogen", "acyl or sulfonyl substituted hydroxy", "acyl or sulfonyl substituted amino" and "alkoxy" as used herein, reference is made to the explanations under item (20) above.
   As to the meaning of the term "desmethyl impurity" as used herein, reference is made to the explanations under item (41) above.
(43) The compound according to any one of items (38) to (42), wherein R₁ is C₁-C₃ alkyl, preferably R₁ is methyl (Me).
(44) A process for producing anacetrapib, comprising the steps of:
   a) providing compound of formula II 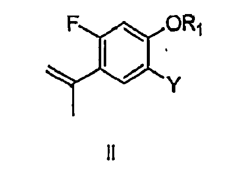 , wherein R₁ is methyl and Y is Cl, Br or I,
      by a process according to any one of items (1) to (9), and
   b) subjecting compound of formula II to further synthesis steps to yield anacetrapib.
(45) Use of compound of formula II 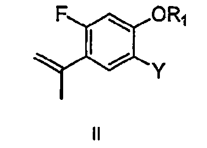 , wherein R₁ is substituted or unsubstituted C₁-C₆ alkyl and Y is Cl, Br or I,
   in a process for preparing a pharmaceutically active agent.
   The term "pharmaceutically active agent" as used herein means any active pharmaceutical ingredient intended for treatment or prophylaxis of a disease of a mammal. In general it means any active pharmaceutical ingredient that has an effect on the physiological conditions of a mammal.
   According to a preferred embodiment, the used compound of formula II has a content of desmethyl impurity in form of 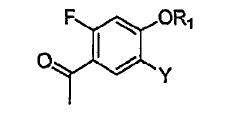 , wherein R₁ and Y are define as above,
   of less than 0.50% by weight, preferably less than 0.25 %, and more preferably less than 0.15 %.
(46) Use of a compound of formula V 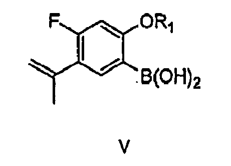 , wherein R₁ is substituted or unsubstituted C₁-C₆ alkyl,
   in a process for preparing a pharmaceutically active agent.
   As to the meaning of the term "pharmaceutically active agent", reference is made to item (45) above.
   According to a preferred embodiment, the used compound of formula V has a content of desmethyl impurity in form of 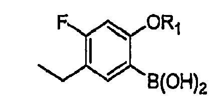 , wherein R₁ is defined as above, of less than 0.50% by weight, preferably less than 0.25 %, and more preferably less than 0.15 %.
(47) Use of a compound of formula VII₂, 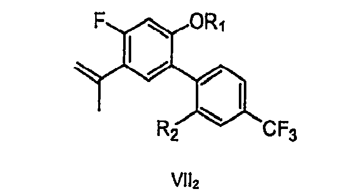 wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro, and R₃ is selected from Isopropyl and 2-propenyl,
   in a process for preparing a pharmaceutically active agent.
   As to the meaning of the terms "leaving group", "halogen", "acyl or sulfonyl substituted hydroxy", "acyl or sulfonyl substituted amino" and "alkoxy" as used herein, reference is made to the explanations under item (20) above.
   As to the meaning of the term "pharmaceutically active agent", reference is made to Item (45) above.
   According to a preferred embodiment, the used compound of formula VII is free of siloxane impurity and has a content of desmethyl impurity in form of 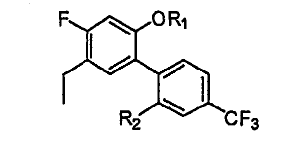 , wherein R₁, R₂ and R₃ are defined as above,
   of less than 0.50% by weight, preferably less than 0.25 %, and more preferably less than 0.15 %.
(48) Use of a compound of formula VII₁ 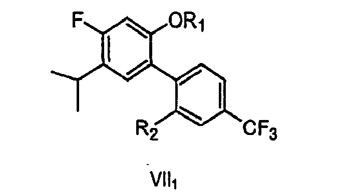 , wherein R₁ is substituted or unsubstituted C₁-C₆ alkyl, and R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro even more preferably R₂ is cyano, hydroxymethyl, formyl, nitro, aminomethyl,
   wherein compound of formula VII₁ is free of siloxane impurity and a content of desmethyl impurity in the compound is less than 0.50% by weight, preferably less than 0.25 % by weight, and more preferably less than 0.15 % by weight.
   in a process for preparing a pharmaceutically active agent.
   As to the meaning of the terms "leaving group", "halogen", "acyl or sulfonyl substituted hydroxy", "acyl or sulfonyl substituted amino" and "alkoxy" as used herein, reference is made to the explanations under item (20) above.
   As to the meaning of the term "pharmaceutically active agent", reference is made to item (45) above.
(49) The use according to any one of items (46) to (48), wherein the pharmaceutically active agent is a compound of formula IX, 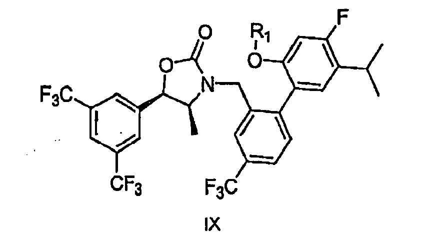 , wherein R₁ is substituted or unsubstituted C₁-C₆ alkyl,
   preferably the pharmaceutically active agent is anacetrapib.
(50) A process for preparing a pharmaceutical composition comprising anacetrapib as a pharmaceutically active agent, comprising the steps of:
   i) preparing anacetrapib according to a process as defined in item (44), and
   ii) admixing the thus prepared anacetrapib with at least one pharmaceutically acceptable excipient.
   The term "pharmaceutically acceptable excipient" as used herein means any physiologically inert, pharmacologically inactive material known in the art being compatible with the physical and chemical characteristics of the active agent.
(51) A pharmaceutical formulation comprising anacetrapib as an active ingredient and at least one pharmaceutically acceptable excipient, wherein anacetrapib is substantially free of desmethylanacetrapib and free of siloxane impurities, preferably anacetrapib is free of both desmethylanacetrapib and siloxane impurities.
   The term "substantially free" as used herein means that the content of desmethylanacetrapib impurity is less than 0.15 % by weight, preferably less than 0.10 % by weight, more preferably less than 0.05 % by weight in relation to the total weight of anacetrapib.

### Detailed description of the invention

In the following, the present Invention will be described in more details by referring to further preferred and further advantageous embodiments and examples, which are however presented for illustrative purposes only and shall not be understood as limiting the scope of the present invention.

The present invention provides an industrially applicable, economical and advantageous process for the preparation of intermediates of cholesterylester transfer protein (CETP) inhibitors, preferably anacetrapib. The preparation process according to the invention starts from 2-(2-fluoro-4-methoxyphenyl)propan-2-ol **(FMOL)** or derivatives thereof which are respective readily available, economical commercial starting materials used in the prior art. However, it has been found and shown that following the prior art process for the synthesis of anacetrapib starting from **FMOL** leads to a formation of desmethyl Impurity **(DMAP),** which is hard to remove from the final product.

It was surprisingly found by the present Invention that applying a dehydration reaction to a compound of formula I 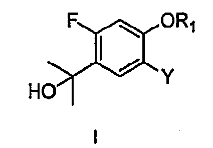 , wherein R₁ is substituted or unsubstituted C₁-C₆ alkyl and Y is Cl, Br or I,
in the presence of a proton donator provides for novel and useful intermediate compounds of formula II 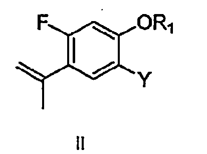 , wherein R₁ and Y are defined as above. Compounds of formula II represent versatile intermediate compounds, which among others can be used in advantageous embodiments to prepare anacetrapib in industry friendly processes requiring lower catalyst loading, milder catalysts, lower pressure and/or shorter reaction times without the need of strong reduction agents. Moreover, the use of compound of formula II as starting compound for the preparation of anacetrapib surprisingly provides for anacetrapib having a significantly higher degree of purity compared to anacetrapib prepared by conventional methods. The process according to the invention allows a route of synthesis which from the outset prevents formation of siloxane and/or desmethyl impurities which are very difficult to remove, or which the conventional art was not aware of.

In the following section, further preferred and further advantageous embodiments and examples are presented for illustrative purposes, wherein these embodiments and examples shall not be understood as limiting the scope of the present invention.

Scheme 8 illustrates a preferred embodiment of the process according to the present Invention wherein compounds of formulae VII₁, and VII₂ in which R₁ is methyl (Me) are prepared, among others via intermediate compound of formula II' **(BrMIPEN)** wherein R₁ is methyl and Y is Br. However, it is understood that this process is also applicable to compounds of formulae I to VII having other substituents R₁ and Y than shown in Scheme 8. 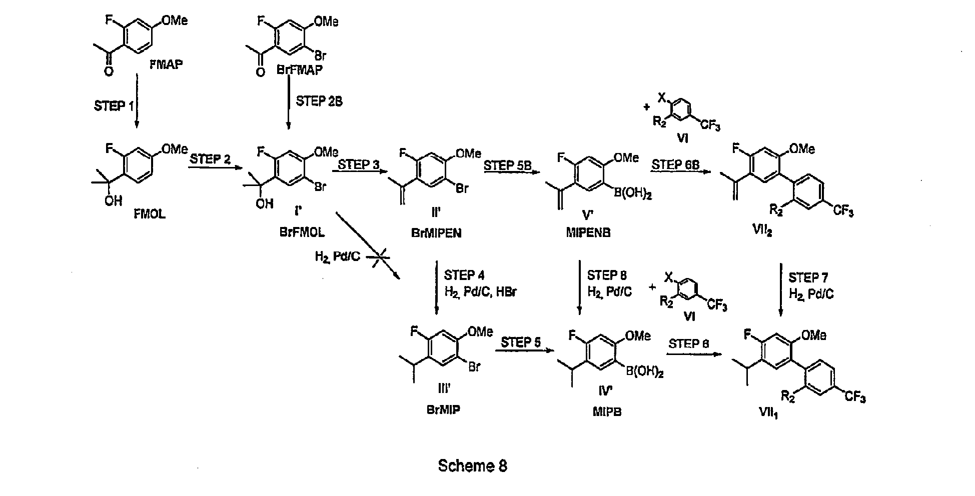 In the first step of the embodiment In Scheme 8, **FMAP** is converted to **FMOL** by treating with MeMgCl in tetrahydrofuran. The crude product **FMOL** contaminated with 5-10 % of starting material **FMAP** is converted to **BrFMOL** using bromination agents such as N-bromosuccinimide, 1,3-dibromo-5,5-dimethylhidantoin (DBDMH) or the like in C₁-C₆ alcohols, acetonitrile or the like with or without water as a co-solvent. The crude **BrFMOL** is optionally crystallized from C₅-C₇-alkane, preferably hexane or ethers, more preferably diisopropyl ether to remove the impurities, especially impurity **FMAP.** Typically, one crystallisation of crude product **BrFMOL** contaminated with 5-10 % of **FMAP** from diisopropyl ether is sufficient to obtain pure **BrFMOL.**

Alternatively, pure **BrFMOL** is prepared from **BrFMAP,** which can e.g. be synthesized according to patent application WO 2007/136672, by Grignard reaction with MeMgCl in tetrahydrofuran. However, in the Grignard reaction according to WO 2007/136672, conversion is not complete and more than 2 % of the starting material **BrFMAP** is detected after isolation. It was surprisingly found that addition of a co-solvent selected from the group consisting of dichloromethane (DCM), toluene or 1,2-dimethoxyethane (DME) to the grignardation reaction mixture provides for an increased conversion of **BrFMAP** to **BrFMOL,** wherein the thus obtained **BrFMOL** was substantially free of the starting material **BrFMAP.**

The Grignard reaction is preferably carried out under an inert atmosphere, for example under an argon or nitrogen atmosphere. The solution of acetophenone **BrFMAP** in DCM, toluene, DME or a mixture of the aforementioned solvents is added to the solution of MeMgCl in tetrahydrofuran, wherein the temperature is maintained below 20 °C, preferably below 5°C. The Grignard reagent is used in an amount of 1.0 to 2.0 equivalents relative to **BrFMAP,** preferably 1.2 to 1.5 equivalents. The reaction is quenched with a C₁-C₆ alcohol, preferably methanol, followed by addition of water and / or acidic water solutions, wherein the temperature is maintained below 20 °C.

The term "quenching" used herein means decomposing a reactive species in order to stop a reaction and to convert intermediate products to stable materials which can be isolated or removed without danger.

In the third step of the embodiment in Scheme 8, **BrMIPEN** is prepared by dehydration of **BrFMOL** In the presence of a proton donator. Preferably, the proton donator is selected from organic or inorganic acids, preferably inorganic acids, more preferably inorganic acids selected from the group consisting of HCl, H₂SO₄, H₃PO₄ and NaHSO₄, and in particular the proton donator is H₂SO₄. Furthermore, the dehydration reaction is preferably carried out in the presence of water. Besides, optionally a solvent such as ethylene glycol, sulfolane, DMSO or polyethylene glycol is added in order to enhance the dehydration rate and /or distillation of **BrFMOL.** According to a further preferred embodiment, dehydration of **BrFMOL** to **BrMIPEN** and isolation of pure **BrMIPEN** are performed simultaneously, preferably by distilling off the reaction products water and/or **BrMIPEN** from the reaction mixture, wherein the reaction mixture is advantageously heated at the temperature of the steam distillation of water and product **BrMIPEN.** For example, steam distillation can be carried out by using a Dean-Stark apparatus. At the end of the distillation step, the pure product **BrMIPEN** can be easily collected by phase separation from water and subsequent drying.

In the fourth step of the embodiment in Scheme 8, **BrMIPEN** is reduced to the key intermediate **BrMIP** by applying hydrogen, Pd/C and minor amounts of HBr in MeOH. The hydrogenation reaction is preferably carried out in the presence of hydrogen, preferably under 15-100 PSI, palladium catalyst such as palladium on activated charcoal, and HBr In solvents, preferably in protic solvents such as MeOH, EtOH, iPrOH or the like at temperatures 10 to 50 °C, preferably at room temperature. Surprisingly, the presence of HBr inhibits the debromination of the substrate **BrMIPEN** and/or product **BrMIP** and could not be substituted with other acids such as HCl. In an alternative embodiment of debromination inhibition, a sulfur compound such as thiophene is added in order to poison the catalyst applied.

**BrMIP** according to this process is preferably substantially free of desmethyl impurities (in the present example, desmethyl impurity is **BrMET**) and free of siloxanes, more preferably **BrMIP** is free of desmethyl impurities and free of siloxanes.

In the fifth step of the embodiment in Scheme 8, **BrMIP** is treated with trialkyl borate, preferably tritsopropyl borate in the presence of an organolithium reagent in an inert aprotic solvent or mixture of solvents to obtain intermediate boric esters, which are further quenched with diluted acid water solution to give a compound of formula **MIPB. MIPB** is then converted to biphenyl **VII₁** by Suzuki coupling with a commercially available or in house prepared compound of formula Vi.

Alternatively, **MIPB** may be prepared from **BrMIPEN** following steps 5B and 8 of the embodiment in Scheme 8. First, **BrMIPEN** is converted to **MIPENB** under the conditions described for Step 5 and then reduced to **MIPB** applying catalytic hydrogenation. Furhermore, **MIPENB** can be directly coupled with the compound **VI** to afford biaryl **VII₂** as shown by examples.

The biaryl compound **VII₁** may be converted further and coupled with the heterocycle of formula **VIII** 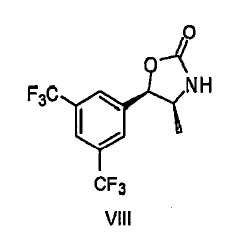 to form anacetrabip of formula **IX** 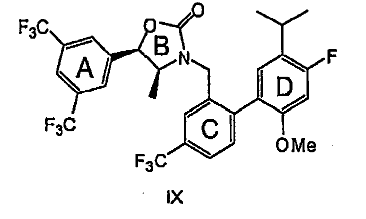 , wherein the capitals A, B, C and D merely indicate the four different ring systems constituting the structure of anacetrabip.

In order to apply the compounds of formula **VII₁** in the synthesis of inhibitors of cholesteryl ester transfer protein (CETP), R₂ should be converted to a group which which provides for a coupling/building to the ring system AB of formula **IX.** For example, a hydroxymethyl group can be converted to chloromethyl or bromomethyl by treating with a corresponding sulphur, phosphorus, oxalyl halide, phosgene derivative or hydrohalic acid or it is sulfonated to give a leaving sulfonyloxy group. A cyano group can be converted to aminomethyl group by reduction, preferably by hydrogenation on Raney Ni and further to the sulfonamide leaving group. An alkoxymethyl such as methoxymethyl group can be demethylated and debenzylated with boron bromides, aluminium bromides or hydrobromic acid to give bromomethyl substituted compounds. An alkoxycarbonyl or formyl group can be reduced to the hydroxymethyl group, which is further transformed as mentioned above.

According to a preferred embodiment, group R₂ of compound of formula **VII₂** is selected such that R₂ can be reduced simultaneously with the 2-propenyl group of formula **VII₂.** This approach is accomplished by selecting a reduction process which is able to simultaneously reduce both groups. For example, compound of formula **VII₂** wherein R₂ is cyano or formyl is reduced simultaneously in a single step reaction to give compound of formula **VII₁** wherein R₂ is CH₂NH₂ or CH₂OH, respectively. The reduced group R₂ can then be converted to a structure which could be directly built to the ring system AB of formula IX as described above. This approach requires one reduction step less than a reaction pathway wherein R₂ group and 2-propenyl group are subsequently reduced, and therefore, it is advantageous in view of yield and time consumption for preparation (cf. Examples 8 in the following experimental part).

Another preferred embodiment of the process according to the present invention is illustrated in Scheme 9 wherein compounds of formulae **VII'₁** and **VII'₂** in which R₁ is methyl (Me) are prepared, wherein preparation starts with compounds of formula II' and III' **(BrMIPEN** and **BrMIP)** wherein R₁ is methyl and Y is Br. However, it is understood that this process is also applicable to compounds of formulae II to VII having other substituents R₁ and Y than shown in Scheme 9.

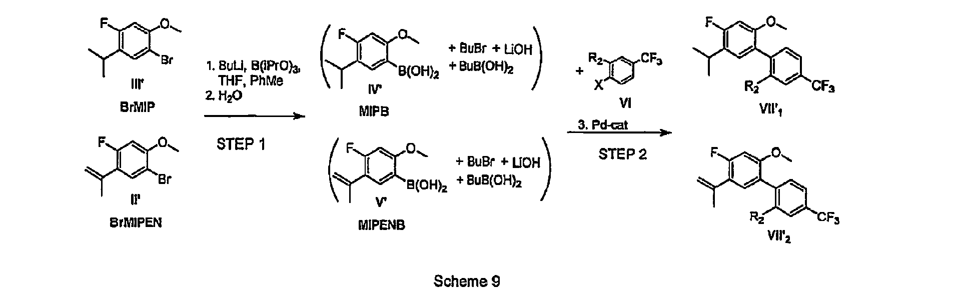

The reactions of the first and the second step of this embodiment are preferably carried out as a one pot conversion. In the first step of this embodiment, **BrMIP** or **BrMIPEN** is treated by trialkyl borate, preferably triisopropyl borate in the presence of an organolithium In an inert aprotic solvent or mixture of solvents to obtain intermediate boric esters, which are further quenched with water to give a compound of formula MIPB or MIPENB or its corresponding salt. In the second step, the resulting mixture from the first step is directly coupled with compound **VI** wherein R₂ is as defined in the above items to obtain compound of formula **VII₁** or **VII₂** under standard Suzuki coupling conditions as shown in the examples.

The person skilled In art would not take into account the aforementioned one pot reaction, since impurities in form of side products such as butylbromide and butylboronic acid forming in the first step are not removed before carrying out Suzuki coupling. Furthermore, under the conditions of the second step, the person skilled in the art would expect problems arising from Heck coupling reaction on 2-propenyl group of **BrMIPEN** competiting with Suzuki coupling. Surprisingly, the aforementioned prejudices could be overcome, and thus none of the aforementioned impurities resulting from step 1 and 2 were formed during this one pot process compared to conventional two step process as shown by examples. Thus, the one pot process makes it possible to avoid the tedious isolation of boronic acid **MIPB** or **MIPENB,** while the number of reaction steps is decreased. That is, this one pot process according to the present invention is significantly economical compared to conventional prior art processes.

According to a further preferred embodiment of the invention, **BrMIP** or **BrMIPEN** is treated with triisopropyl borate, preferably 1.0 to 2.0 equivalents relative to **BrMIP** or **BrMIPEN** in the presence of 1.0 to 1.5 equivalents of BuLi to limit the formation of BuB(OH)₂ in toluene/tetraydrofuran mixture or the like, maintaining the temperature below -25 °C, preferably below -50 °C. Then, water is added to prepare in situ boronic acid **MIPB or MIPENB,** respectively, and base (LiOH), followed by addition of Pd-ligand catalyst system, preferably Pd(OAc)₂/PPh₃ or 1,1, bis(di-tertbutylphosphino)ferrocene palladium dichloride. The resulting mixture is aged from 15 °C to the reflux temperature, preferably from 25 °C to 50 °C, to obtain compound **VII'₁** or **VII'₂,** respectively. Alternatively, aryl lithium intermediate of **BrMIP** or **BrMIPEN** in the preparation of corresponding boronic acids may be prepared by treating **BrMIP** or **BrMIPEN** with lithium in C₅-C₇-alkane or the like.

In the following, embodiments (A) to (G) are elucidated in order to exemplify particularly preferred routes for preparing anacetrapib or intermediate compounds for the synthesis of anacetrapib. In the illustrative and preferred embodiments shown in the following, compounds of formulae I' to VII' wherein R₁ = methyl (Me) and Y = Br are depicted as the starting materials. However, it is understood that the following processes are also applicable to compounds of formulae I to VII having substituents R₁ and Y other than shown in the following exemplary embodiments.

According to embodiment (A), the synthesis of a compound of formula IX, 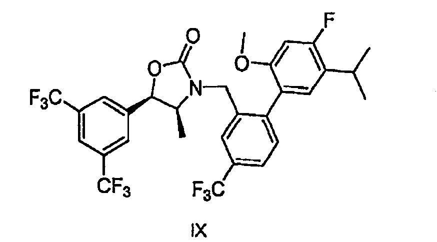 comprises
a) dehydration of the compound of formula I' (BrFMOL) 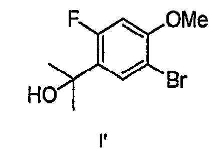 to a compound of formula II' (BrMIPEN) 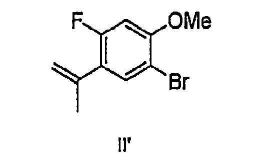 In the presence of a proton donator;
b) hydrogenating the compound of formula II' (BrMIPEN) to obtain the compound of formula III' (BrMIP) 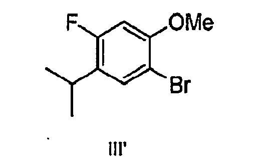 preferably in the presence of palladium catalyst and HBr or in the presence of palladium catalyst and thiophen in C₁-C₆-alcohol solution;
c) converting the compound of formula III' **(BrMIP)** to a compound of formula IV' **(MIPB),** 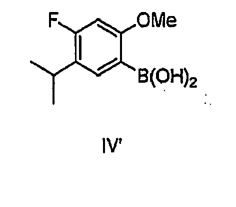 by treating with trialkyl borate in the presence of an organolithium compound in an Inert aprotic solvent or mixture of solvents;
d) coupling the compound of formula IV' (MIPB) with a compound of formula VI, 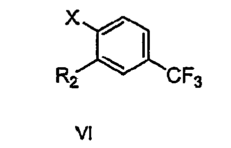 wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   to form a compound of formula VII₁, 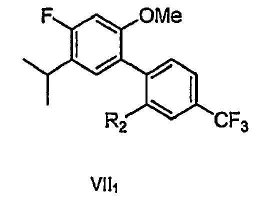 wherein R₂ is defined as above;
e) optionally converting a compound of formula VII₁ to a compound of formula VII₁', 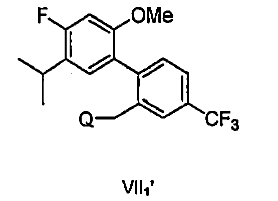 wherein R₂ is CH₂Q in which Q represents a leaving group, preferably Q is selected from the group consisting of halogen, sulfonyl substituted hydroxyl or amino;
f) coupling a compound of formula VII₁' with a compound of formula VIII, 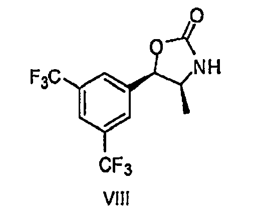 In order to obtain a compound of formula IX, 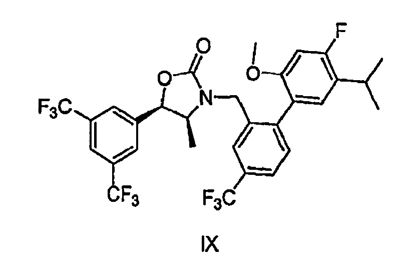
g) optionally purifying the compound of formula IX to reduce the level of by-products, epimers and/or enantiomers.
   According to another embodiment (B), the synthesis of a compound of formula IX is accomplished by replacing steps b) to d) of embodiment (A) by the following steps h) to j):
h) converting a compound of formula II' **(BrMIPEN)** to a compound of formula V' **(MIPENB),** 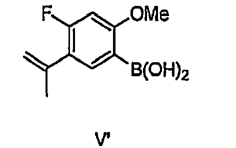 by treating with trialkyl borate in the presence of an organolithium compound in an inert aprotic solvent or mixture of solvents;
i) coupling a compound of formula V' **(MIPENB)** with a compound of formula VI, 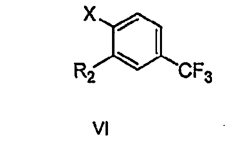 wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   to form a compound of formula VII₂, 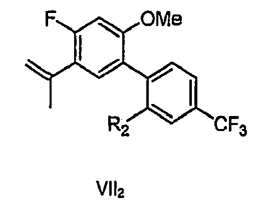 wherein R₂ is defined as above;
j) reduction of the compound of formula VII₂ to form a compound of formula VII₁, 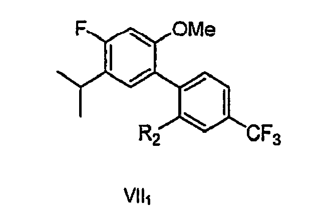 wherein R₂ is the same as above,
   preferably by the catalytic hydrogenation.
   According to embodiment (C), compound of formula IX is prepared according to the process according to embodiment wherein step i) is replaced by step k)
k) reduction of the compound of formula V' **(MIPENB)**
   to form a compound of formula IV' **(MIPB),** 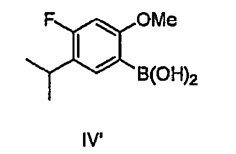 preferably by catalytic hydrogenation,
   and optionally further converting the compound of formula IV' **(MIPB)** according to process steps d) to g) of embodiment (A).
   According to embodiment (D), synthesis of compound of formula IX is accomplished by replacing process steps c) and d) of embodiment (A) by the following process step I) :
l) one pot reaction of the compound of formula III' BrMIP to a compound of formula VII₁ 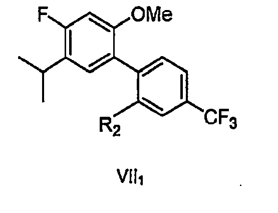 wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   by treating with trialkyl borate in the presence of an organolithium in an inert aprotic solvent or mixture of solvents to give intermediate boric esters, which are further quenched with water to give a compound of formula IV' **(MIPB)**
   followed by addition of compound of formula VI and transition metal with corresponding ligands preferably selected from palladium-triarylphosphine, -trialkylphosphine or -aryl and alklyl substituted phosphine complexes with palladium in the zero oxidation state, salts of palladium in the presence of phosphine ligands, and metallic palladium optionally supported on a solid.
   According to embodiment (E), synthesis of compound of formula IX is accomplished by embodiment (B) wherein step h) is replaced by the following step m):
m) one pot reaction of the compound of formula III' **(BrMIPEN)** to a compound of formula VII₂, 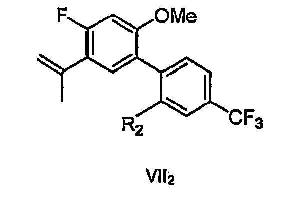 wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
   by treating with trialkyl borate in the presence of an organolithium in an inert aprotic solvent or mixture of solvents to give intermediate boric esters, which are further quenched with water in order to obtain a compound of formula V' (**MIPENB**)
   followed by addition of compound of formula VI and transition metal with corresponding ligands preferably selected from palladium-triarylphosphine, -trialkylphosphine or-aryl and alklyl substituted phosphine complexes with palladium in the zero oxidation state, salts of palladium in the presence of phosphine ligands, and metallic palladium optionally supported on a solid.

According to embodiment (F), the starting compound of step a) of embodiment (A), that is compound of formula I' **(BrFMOL)** 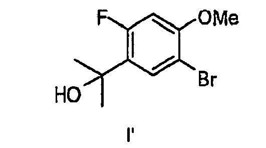 is prepared by bromination of compound of formula X' (FMOL) 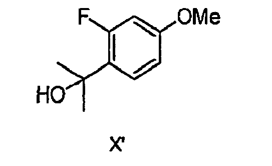 with bromination reagents and
optionally purifying compound of formula X, preferably by re-crystallization.

According to embodiment (G), starting compound of step a) of embodiment (A), that is compound of formula I' **(BrFMOL)** 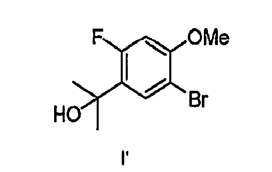 is prepared by treating a compound of formula XI' **(BrFMAP)**, 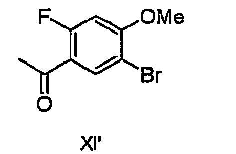 with a methylmagnesium halide in the presence of a co-solvent selected from the group consisting of dichloromethane, toluene or 1,2-dimethoxyethane.

The following examples further illustrate the invention. They are provided for illustrative purposes only and are not intended to limit the invention in any way. The examples and modifications or other equivalents thereof will become apparent to those versed in the art in the light of the present entire disclosure

### Experimental procedures

### Comparative example 1: Synthesis of biphenyl VII according to patent applicationWO 2007/005572

### Step 1: Synthesis of 2-(2-fluoro4-methoxyphenyl)propan-2-ol (compound of formula 2, Scheme 3)

The commercially available 3M solution of MeMgCl (400 mL, 1.2 mol) in THF was cooled to -15 °C, and a solution of 1-(2-fluoro-4-methoxyphenyl)ethanone (compound of formula 1, Scheme 3) (118 g, 0.7 mol) in dry THF (400 mL) was added dropwise, maintaining the internal temperature below 0 °C. The reaction mixture was then aged at temperature -5 to 0 °C for an hour. The reaction was quenched with 2M HCl (700 mL) in a dropwise fashion maintaining, the internal temperature below 15 °C. The quenched reaction was then aged at 10 °C for 1 hour and extracted with petrol ether. The organic phase was then washed with water (2×700 mL) and the solvent removed under reduced pressure to yield a slightly bluish oil (121.8 g, 95%).
¹H NMR (CDCl₃) δ 1.61 (d, *J* = 1.0 Hz, 6H), 2.12 (bs, 1H), 3.78 (s, 3H), 6.61 (dd, *J₁* = 20.6 Hz, *J₂* = 2.7 Hz, 1H), 6.65 (dd, *J₁* =15.2 Hz, *J₂* = 2.5 Hz, 1H), 7.43 (dd, *J₁* = 9.5 Hz, *J₂* = 8.7 Hz, 1H).

The product was contaminated with -5 % of the starting material 1. The reaction was repeated with CeCl₃ according to patent application WO 2007/005572. CeCl₃ should prevent enolization of starting material and thus should give a quantitative reaction yielding pure product **2** without starting material **1**. However, the result were even poorer regarding the remaining starting material in the product **2** and also other impurities were detected.

### Step 2: Synthesis of 2-fluoro-1-isopropyl-4-methoxybenzene (compound of formula 3, Scheme 3)

To a solution of compound of formula **2** (Scheme 3) (4.20 g, 25 mmol) in EtOH (30 mL), 37 % HCl (2.2 mL, 27 mmol) diluted with EtOH (0.5 mL) was added, followed by 10% Pd/C (50% water) (66 mg, 0.5 mol%). The mixture was placed under 15 psi hydrogen at 40 °C until the reaction was complete based on HPLC analysis for an approximately 1 hour. The mixture was cooled to room temperature and the catalyst was removed by filtration. The solvent was removed under reduced pressure to a half of volume and water (10 mL) was added. The product of formula 3 was extracted into petrol ether and the solvent removed under the reduced pressure to yield anisole of formula 3 as a colorless oil (4.14 g, 82%).
¹H NMR (CDCl₃) δ 1.22 (d, *J* = 6.9 Hz, 6H), 3.15 (sept., *J* = 6.9 Hz, 1H), 3.76 (s, 3H), 6.58 (dd, *J₁* = 21.6 Hz, *J₂* = 2.6 Hz, 1H), 6.62 (dd, *J₁* = 15.2 Hz, *J₂* = 2.5 Hz, 1 H), 7.12 (t, *J* = 8.7 Hz, 1H).

The impurity 1-ethyl-2-fluoro-4-methoxybenzene (MET) (~5%), which is formed from 1-(2-fluoro-4-methoxyphenyl)ethanone present in the starting material under the conditions described in Step 2, was detected in the product

### Step 3: Synthesis of 1-bromo-4-fluoro-5-isopropyl-2-methoxybenzene (compound of formula 4, Scheme 3)

2-fluoro-1-isopropyl-4-methoxybenzene (compound of formula 3, Scheme 3) (34.3 g, 0.204 mol) was dissolved in acetonitrile (400 mL). The solution was warmed to 35 °C, and NBS (40 g, 0.225 mol) was added in a single solid addition. The reaction was maintained at 35°C and was completed In 3-4 hours. The reaction was quenched with 2M Na₂SO₃ (40 mL, 0.08 mol). The resulting mixture was concentrated to ¼ of the total volume and diluted with water (400 mL) and petrol ether (300 mL). The organic layer was cut and washed with water (300 mL), dried over Na₂SO₄ and filtered. The solvent was removed under reduced pressure to yield 1-bromo-4-fluoro-5-isopropyl-2-methoxybenzene (compound of formula 4, Scheme 3) as a slightly yellow oil (49.26 g, 98%).¹H NMR (CDCl₃) δ 1.22 (d, *J* = 6.9 Hz, 6H), 3.14 (sept, *J* = 6.9 Hz, 1H), 3.85 (s, 3H), 6.60 (d, *J* = 11.8 Hz, 1H), 7.37 (d, *J* =8.0 Hz, 1H).

The impurity 1-bromo-5-ethyl-4-fluoro-2-methoxybenzene (BrMET) (~5%), which is formed from 1-ethyl-2-fluoro-4-methoxybenzene (MET) present in the starting material under the conditions described in Step 3, was detected In the product.

### Step 4: Synthesis of 4-fluoro-5-isopropyl-2-methoxyphenylboronic acid (compound of formula 5, Scheme 3)

A 500 mL dry flask was charged with 2-bromo-5-fluoro-4-isopropylanisole (compound of formula 4, Scheme 3) (24.6 g, 0.1 mol) and dissolved in toluene (80 mL) and THF (80 mL). The resulting solution was flushed with argon, and tri-isopropylborate (32 mL, 0.14 mol) was added. The mixture was cooled to -80 °C. Then 10 M n-BuLi in hexanes (12.5 mL, 0.125 mol) was added slowly, maintaining a temperature below -55°C. Thirty minutes after completion of the n-BuLi addition, the reaction was warmed to -35°C and quenched into 3 M H₂SO₄ solution (75 mL, 0.225 mol). DIPE (200 mL) was added to the mixture to dilute the organic layer. The mixture was stirred (15 min) and the aqueous layer was cut away. The organic layer was washed with 3.0 M H₂SO₄ (75 mL). The organic phase was extracted three times with 1 M NaOH (200 mL first and then 50 mL and 50 mL). The three NaOH extractions were combined, diluted with 2-propanol (85 mL), and cooled to 15 °C. Then the solution was slowly acidified to pH - 2 using 3 M H₂SO₄ (70 mL) while maintaining temperature at 15-20 °C. The resulting slurry was stirred for 1 hour and then filtered. The filter cake was washed with water (3 x 30 mL) and dried under an air flow for 1 day. The filtered solid was placed in an oven under vacuum at 50 °C for 2-3 days to decompose a diaryl impurity and to dry the solid. The white crystalline solid was isolated to yield boronic acid of formula 5 (Scheme 3) (19.23 g, 91%): mp 100-102 °C; ¹H NMR (CDCl₃) δ 1.25 (d, *J* = 6.9 Hz, 6H), 3.17 (sept., *J* = 6.9 Hz, 1H), 3.88 (s, 3H), 5.83 (s, 2H), 6.59 (d, *J* = 12.4 Hz, 1 H), 7.72 (d, *J* = 6.6 Hz, 1H).

The impurity 5-ethyl-4-fluoro-2-methoxyphenylboronic acid (~4%), which is formed from 1-bromo-5-ethyl-4-fluoro-2-methoxybenzene **(BrMET)** present in the starting material under the conditions described in Step 4, was detected in the product.

### Step 5 (Suzuki reaction): Synthesis of (4'-fluoro-5'-isopropyl-2'-methoxy-4-(trifluoromethyl)biphenyl-2-yl)methanol (compound of formula 6, Scheme 3)

A 3 M K₂CO₃ solution is prepared by adding solid K₂CO₃ (31 g, 0.22 mol) to water (100 mL). Cooling is applied to keep the solution at 20-25 °C. (2-chloro-5-(trifluoromethyl)phenyl) methanol (compound of formula **13**) (17.5 g, 84 mmol), and boronic acid 5 (Scheme 3) (18.1 g, 85 mmol) are added to the K₂CO₃ followed by THF (100 mL) rinse. The solution is degassed by sparging with argon gas for 20 min. The catalyst, 1,1 bis(di-tert-butylphosphino)ferrocene palladium dichloride (300 mg, 0.55 mol%) is added. The organic layer turns dark brown immediately. The biphasic mixture is aged at 35-45 °C with vigorous stirring for 32 hours. The mixture is cooled to room temperature and water (150 mL) is added, followed by petrol ether (150 mL) and the aqueous layer is removed. The organic layer was washed with water (2×200 mL) and filtered through silica gel and the solvent is removed under reduced pressure to yield brownish oil which is crystallized from heptane to give a pale white solid (28.5 g, 80%). mp 93.5-95.5 °C; ¹H NMR (CDCl₃) δ 1.24 (d, *J* = 6.9 Hz, 6H), 1.95 (t, *J* = 6.1 Hz, 1H), 3.21 (sept., *J* = 6.9 Hz, 1 H), 3.73 (s, 3H), 4.49 (m, 2H), 6.68 (d, *J* =12.0 Hz, 1 H), 6.99 (d, *J* = 8.6 Hz, 1 H), 7.30 (d, *J* = 7.9 Hz, 1H), 7.59 (dd, *J₁* = 8.0 Hz, *J₂* =1.3 Hz, 1 H), 7.86 (d, *J* = 0.7 Hz, 1H).

The impurity (5'-ethyl-4'-fluoro-2'-methoxy-4-(trifluoromethyl)biphenyl-2-yl)methanol (- 3 %), which is formed from 5-ethyl-4-fluoro-2-methoxyphenylboronic acid present in the starting material under the conditions described in Step 5, was detected in the product.

### Step 6: Synthesis of 2'-(chloromethyl)-4-fluoro-5-isopropyl-2-methoxy-4'-(trifluromethyl)biphenyl (compound of formula 7, Scheme 3)

To a solution of biaryl compound 6 (Scheme 3) (28.5 g, ∼ 75 mmol) in DMF (140 mL) which was maintained at 10 °C was added thionyl chloride (7.3 mL, 100 mmo), and then the mixture was warmed to room temperature. The mixture was aged for 5 hours and water (200 mL) was then added. The crystallization described in the prior art from DMF/water could not be repeated so that is why the product was extracted with petrol ether (150 mL). The organic phase was washed with water (3x150 mL) and the solvent removed under reduced pressure to obtain a brown oil (29.2 g) which was then crystallized from MeOH (60 mL). The solid was filtered and washed with ice cold MeOH (20 mL) to yield a compound of formula 6 (Scheme 3) as colorless crystals (20.95 g, 78% for two steps): mp 45-49 °C; ¹H NMR (CDCl₃) δ 1.25 (d, *J* = 6.9 Hz, 6H), 3.22 (sept., *J* = 6.9 Hz, 1H), 3.72 (s, 3H), 4.43 (dd, *J₁* = 34.6 Hz, *J₂* =10.0 Hz, 2H), 6.68 (d, *J* =12.0 Hz, 1H), 7.08 (d, *J* =8.6 Hz,1H), 7.33 (d, *J* = 8.0 Hz, 1H), 7.60 (dd, *J₁* = 8.0 Hz, *J₂* = 1.3 Hz, 1H), 7.83 (d, *J* = 0.7 Hz, 1H).

The impurity 2'-(chloromethyl)-5-ethyl-4-fluoro-2-methoxy-4'-(trifluoromethyl)biphenyl (**EBFCI**) (- 3 %), which is formed from (5'-ethyl-4'-fluoro-2'-methoxy-4-(trifluoromethyl)biphenyl-2-yl)methanol present In the starting material under the conditions described in Step 6, was detected in the product.

### Step 7: Synthesis of (4S,5R)-5-[3,5-bis(trifluoromethyl)phenyl]-3-[[2-(4-fluoro-2-methoxy-5-propan-2-ylphenyl)-5-(trifluoromethyl)phenyl]methyl]-4-methyl-1,3-oxazolidin-2-one (anacetrapib)

The chiral intermediate (4S,5R)-5-(3,5-bis(trifluoromethyl)phenyl)-4-methyloxazolidin-2-one (compound **11** in Scheme 3, prepared by procedure of WO 2007/005572) (28.0 g) is dissolved in DMF (300 mL) and cooled to - 15°C. 2 M NaHMDS (39.2 mL, 1.05 eq) was then added over 1 h, followed by addition of the biaryl chloride 7 (Scheme 3) (28.0 g) in DMF (50 mL), maintaining the internal temperature below -10 °C. The mixture was warmed to + 12 °C and was aged until complete conversion took place. Then 5M HCl (35 mL) was added, followed by 160 mL of 10% IPAC/Heptanes and 340 mL of water, keeping the temperature between 10°C and 20°C throughout. The layers were cut and the organic layer was washed twice with 150 mL of 1/1 DMF/water followed by two 140 mL waterwashes. The organic layer was then removed under reduced pressure and the resulting residue was purified by flash chromatography (EtOAc/hexanes) to remove the excess oxazolidinone **11** (Scheme 3). The obtained colorless oil was then dissolved in refluxing heptanes (200 mL) and the solution was slowly cooled to -20 °C. The resulting slurry was then stirred at -20 °C for 2 hours and filtered. The filter cake was washed with cold heptanes and was then dried, yielding 44.0 g (88%) of the desired product of Formula **IX** (anacetrapib) as an amorphous material.

The impurity (4S,5R)-5-(3,5-bis(trifluoromethyl)phenyl)-3-((5'-ethyl-4'-fluoro-2'-methoxy-4-(trifluoromethyl) biphenyl-2-yl)methyl)-4-methyloxazolidin-2-one (**DMAP**) (∼3%), which is formed from 2'-(chloromethyl)-5-ethyl-4-fluoro-2-methoxy-4'-(trifluoromethyl)biphenyl (**EBFCI**) present in the starting material under the conditions described in Step 7, was detected in the product.

### Comparative example 2: Synthesis of biphenyl VII according to patent application WO 2007/136672

### Step 1: Synthesis of 1-bromo-4-fluoro-2-methoxybenzene (compound of formula 2, Scheme 6)

A mixture of 1-bromo-2,4-difluorobenzene **1** (Scheme 6) (75.6 g, 0.39 mol), KOMe (37 g, 0.5 mol) in THF (300 mL) was heated to 65 - 68°C and stirred at that temperature for 5 hours. The reaction mixture was cooled to room temperature and water (300 mL). The resulting mixture was extracted with DIPE and the organic layer was washed twice with water (2 x 150 mL) and brine (100 mL), dried over Na₂SO₄ and filtered. The solvent was removed under reduced pressure to give coloriess oil which was distilled under reduced pressure (90-92 °C, 5 mbar) to yield anisole of formula **2** (Scheme 6) as a coloriess oil (74.4 g, 93%): ¹H NMR (CDCl₃) δ 3.88 (s, 3H), 6.54-6.68 (m, 2H), 7.47 (dd, *J₁* = 8.7 Hz, *J₂* = 6.2 Hz, 1H).

### Step 2: Synthesis of 1-(5-bromo-2-fluoro-4-methoxyphenyl)ethanone (compound of formula 3, Scheme 6)

The solution of anisole of formula **2** (Scheme 6) (61.5 g, 0.3 mol) in 1,2-dichloroethane (DCE) (600 mL) was cooled to -10 °C and AlCl₃ was added in portions, maintaining the temperature below 2 °C. The solution was then cooled to -5 °C and acetyl chloride (56 mL, 0.79 mol) was added dropwise maintaining the internal temperature below 5 °C. The reaction mixture was aged for 1 hour at 0 °C. At this time HPLC showed no more starting material. The reaction mixture was quenched into ice (600 g) at a rate such that the internal temperature remained below 20 °C. The resulting mixture was extracted with EtOAc (400 mL) and the organic phase then washed with 1 M HCL (400 mL), saturated NaHCO₃ (400 mL) and brine (200 mL), dried over Na₂SO₄ and filtered. The solvent was removed under reduced pressure to give acetophenon of formula 3 (Scheme 6) as a white solid (74.0 g,100%): mp 113-117 °C; ¹H NMR (CDCl₃) δ 2.59 (d, *J* = 5.1 Hz, 3H), 3.95 (s, 3H), 6.64 (d, *J* = 12.5 Hz, 1H), 8.13 (d, *J* = 7.9 Hz, 1H).

### Step 3: Synthesis of 2-(5-Bromo-2-fluoro-4-metoksifenil)propan-2-ol (compound of formula 4, Scheme 6)

The commercially available 3M solution of MeMgCl (200 mL, 0.6 mol) in THF was cooled to -15 °C, and a solution of 1-(5-bromo-2-fluoro-4-methoxyphenyl)ethanone (compound of formula **3**, Scheme 6) (86.5 g, 0.35 mol) in dry THF (200 mL) was added dropwise, maintaining the internal temperature below 0 °C. The reaction mixture was then aged at temperature -5 to 0 °C for an hour. The reaction was quenched with 2M HCl (350 mL) in a dropwise fashion maintaining the internal temperature below 20 °C. To the resulting mixture EtOAc (500 mL) was added, stirred for 10 min and the agues layer was cut off. The organic layer was washed with brine (200 mL) and a solution of half saturated brine (150 mL brine + 150 mL water). The organic layer was transferred into a flask and concentrated under reduced pressure to a final volume of 350 mL, then solvent switched to hexanes (1500 mL) at this volume. The resulting slurry was cooled to 0 °C and filtered. The obtained solid was washed with the ice cold hexanes (100 mL) and dried overnight under vacuum at 40 °C and 10 mbarto yield carbinol of formula **4** (Scheme 6) as a white solid (79.2 g, 88%). mp 75-86 °C; ¹H NMR (CDCl₃) δ 1.606 (s, 6H), 2.02 (s,1H), 3.87 (s, 3H), 6.62 (d, *J* = 13.2 Hz, 1H), 7.73 (d, *J* = 8.7 Hz, 1H).

The product was contaminated with ∼2 % of starting material 3 (**Scheme 6**).

### Step 4: Synthesis of 1-bromo-fluoro-5-isopropyl-2-methoxybenzere (compound of formula 5, Scheme 6)

To a flask equipped with a magnetic stirrer, a nitrogen inlet and a temperature probe was charged with tetramethyldisiloxane (17 mL, 0.1 mol) and 1,2-dichloroethane (65 mL), cooled to -20°C, and then TFA (10 mL) was added. Then, a solution of carbinol of formula **4** (Scheme 6) (26.3 g, 0.1 mol) in DCE (90 mL) was added dropwise, maintaining the internal temperature below-10 °C. The reaction mixture was aged for 15 min at-10 °C. The reaction mixture was transferred into saturated NaHCO₃ (120 mL), stirred for 5 min, and the layers were cut. The bottom organic layer was washed twice with saturated NaHCO₃ (2×100 mL) and brine (50 mL), dried over Na₂SO₄ and finally filtered. The solvent was removed under reduced pressure to give brown oil (26.3 g) which was then purified by distillation using 10 cm Vigreux column at 102-104 °C and 5 mbar to yield 5 as a colorless oil (15.2 g, 62 %). ¹H NMR (CDCl₃) δ 1.22 (d, *J*= 6.9 Hz, 6H), 3.14 (sept., *J*= 6.9 Hz, 1H), 3.85 (s, 3H), 6.60 (d, *J* = 11.8 Hz, 1H), 7.37 (d, *J* =8.0 Hz, 1 H).

The product was contaminated with - 5% of siloxanes.

### Step 5: Synthesis of 2-(3-(trifluoromethyl)phenyl)-4,5-dihydrooxazole (compound of formula 7, Scheme 6)

A flask equipped with a magnetic stirrer, a nitrogen inlet, a temperature probe and a condenser was charged with the 3-(trifluoromethyl)benzonitrile (compound of formula **6**) (85.5 g, 0.5 mol), followed by ethanolamine (62 ml, 1 mol), CaCl (9.0 g, 0.075 mol) and xylenes (45 mL). The reaction mixture was heated to 125 °C and stirred at this temperature for 24 hr. Then the reaction mixture was allowed to cool to about 40°C, and transferred with DIPE (350 mL) into an extractor containing 0.1 M KH₂PO₄ (600 mL). The organic phase was cut and washed with water (2×400 mL), brine (200 mL), dried over Na₂SO₄ and filtered. The solvent was then removed under reduce pressure. The obtained crude residue was distilled using 10 cm column with Rashig rings at 90 °C and 5 mbar to yield oxazoline 7 (Scheme 6) as a colorless oil (83.2 g, 77%): ¹H NMR (CDCl₃) δ 4.09 (t, *J* = 9.5 Hz, 2H), 4.47 (t, *J* = 9.5 Hz, 2H), 7.54 (t, *J* = 7.8 Hz, 1H), 7.73 (d, *J* = 7.8 Hz, 1H), 8.13 (d, *J* = 7.8 Hz, 1 H), 8.23 (s, 1H).

### Step 6: Synthesis of 2-(4'-fluoro-5-isopropyl-2'-methoxy-4-(trifluoromethyl)biphenyl-2-yl)-4,5-dihydrooxazole (compound of formula 8, Scheme 6).

A dry flask equipped with a magnetic stirrer, a argon inlet and a temperature probe was charged with oxazoline 7 (22.14 g, 0.10 mol) followed by anisole of formula 5 (Scheme 6) (24.60 g, 0.10 mol) and NMP (125 mL predegassed by bubbling argon for 30 min). Then K₃PO₄ (32.00 g, 0.15 mol) and KOAc (1.60 g, 0.016 mol) were charged. This suspension was degassed for an additional 30 minutes. The reaction mixture was then heated to 130 °C.

A flask was charged with degassed NMP (25 mL) and triphenylphosphine (240 mg), and then was degassed by bubbling argon for 20 minutes at rt. The ruthenium complex dimer (250 mg) was then added, and this solution was degassed for an additional 15 minutes at rt.

When the reaction mixture in the first flask reached 130°C, half of the ruthenium solution (12 mL) was transferred. The reaction temperature was lowered to 110 °C, and the reaction was stirred at this temperature for 2 hours before the addition of the second half of the ruthenium catalyst. The reaction mixture was then stirred at this temperature for 16 hr and monitored by HPLC. It was noticed that the reaction is quick at the beginning and then stops.

The addition of new portion of catalyst (150 mg) and ligand (150 mg) started the reaction again but was then quickly stopped. The reaction mixture was stirred at 120 °C for additional 24 h and then quenched with water maintaining the internal temperature below 25 °C. The product precipitated as an oil and not as solid as reported in prior art. Thus the obtained mixture was extracted with DIPE (500 mL). The obtained organic phase was washed with water (3x250 mL), dried over Na₂SO₄ and active charcoal, and filtered through silica gel pot. The solvent was removed under reduced pressure to give brown oil (34.65 g). The ¹HNMR showed that crude product contains biphenyl **8** (Scheme 6) and starting material **5** (Scheme 6) and **7** (Scheme 6) in the mol ratio - 2:1:1, respectively. The impurities including starting material **5** (Scheme 6) and **7** (Scheme 6) were removed by distillation using 5 cm Vigreux distillation column at 170 °C and 0.15 mbar. The obtained residual brown oil slowly solidified upon standing at room temperature to yield the compound of formula **8** as a brown solid (25.0 g, 65%) which was used in the next step without further purification. ¹H NMR (CDCl₃) δ 1.24 (d, *J* = 6.9 Hz, 6H), 3.20 (sept., *J* = 6.9 Hz, 1H), 3.71 (s, 1H), 3.90 (t, *J* = 9.6 Hz, 1H), 4.15 (t, *J*=9.6 Hz, 1H), 6.61 (d, *J*=12.1 Hz. 1H), 7.06 (d, *J* = 8.6 Hz, 1H), 7.47 (d, *J* = 8.1 Hz, 1H), 7.70 (dd, *J*₁ = 8.1 Hz, *J*₂ =1.4 Hz, 1H), 8.12 (d, *J* = 1.4 Hz, 1H).

### Step 7: Synthesis of (4'-fluoro-5'-isopropyl-2'-methoxy-4-(trifluoromethyl)biphenyl-2-yl)methanol (compound of formula 9, Scheme 6)

A round bottom flask equipped with a magnetic stirrer, a argon inlet, a temperature probe and a condenser was charged with biaryl oxazoline of formula **8** (Scheme 6) (25.0 g, - 0.065 mol) and THF (100 mL). Then diisopropyl ethyl amine (1.15 mL, 10 mol%) followed by methyl chloroformate (5.70 mL, 0.074 mol) were added at room temperature, and the resulting mixture was heated to 62 °C for 45 minutes. HPLC showed complete conversion of the compound of formula 8 (Scheme 6) to the activated intermediate. The reaction mixture was then cooled to 0 °C, and NaBH₄ (7.0 g, 0.185 mol) was added In one portion followed by slow addition of water (25 mL) over 1 hour while maintaining the internal temperature below 20 °C. The reaction mixture was aged at rt for 12h. HPLC showed complete conversion to biaryl alcohol of formula 9 (Scheme 6). The reaction mixture was cooled to 0 °C, and 2 M HCl (100 mL) was added dropwise while maintaining the temperature between 0 °C and 4 °C. The prior art describes the crystallization of the product by treating the obtained mixture with MeOH. This crystallization could not be repeated at this scale. Thus the obtained mixture above was extracted with DIPE (150 mL). The organic layer was then washed with water (200 mL), saturated NaHCO₃ (200 mL), and again with water (200 mL). The solvent was removed under reduced pressure to give brown oil (24.2 g). The oil was dissolved in heptanes (80 mL) and charcoal was added. The mixture was stirred at room temperature for 2 hours and charcoal was then removed by filtration. The heptane solution was cooled to -15 °C. The product precipitated first as an oil and then solidified upon standing at-15 °C in heptane. The solid material was collected and dried to yield a compound of formula **9** (Scheme 6) as a grey solid (19.0 g, ∼ 60% calculated on the pure **9** (Scheme 6) based on ¹HNMR). Also, the HPLC showed two additional peaks with shorter retention time.

### Example 1: Synthesis of 1-bromo-4-fluoro-5-(2-propenyl)-2-methoxybenzene (BrMIPEN)

### Step 1: Synthesis of 2-(2-fluoro-4-methoxyphenyl)propan-2-ol (FMOL)

### The commercially available 3M solution of MeMgCl (200 mL, 0.6 mol) in THF was cooled to -15 °C, and a solution of 1-(2-fluoro-4-methoxyphenyl)ethanone (59 g, 0.35 mol) in dry THF (200 mL) was added dropwise, maintaining the internal temperature below 0 °C. The reaction mixture was then aged at temperature -5 to 0 °C for an hour. The reaction was quenched with 2M HCl (350 mL) in a dropwise fashion maintaining, the internal temperature below 15 °C. The quenched reaction was then aged at 10 °C for 1 hour an extracted with petrolether. The organic phase was then washed with water (2×350 mL) and the solvent removed under reduced pressure to yield a slightly bluish oil (61.0 g, 95%): ¹H NMR (CDCl₃) δ 1.61 (d, J = 1.0 Hz, 6H), 2.12 (bs, 1H), 3.78 (s, 3H), 6.61 (dd, J₁ = 20.6 Hz, J₂ = 2.7 Hz, 1H), 6.65 (dd, J₁ =15.2 Hz, J₂ = 2.5 Hz, 1H), 7.43 (dd, J₁ = 9.5 Hz, J₂ = 8.7 Hz, 1H).

The product contained - 5 % of unreacted starting material FMAP and was used in the next step without further purification.

### Step 2: Bromination of FMOL to BrFMOL

**FMOL** (54.78 g, 297 mmol) from the step 1 was diluted by acetonitrile (300 mL), and N-bromosuccinimide (57.92 g, 327 mmol) was added in one portion. The resulting mixture was heated to 60 °C and was aged until complete conversion took place (2-3 hours). The reaction was quenched with 2M Na₂S₂O₃ (aq) (150 mL) and stirred for another 15 minutes. The solution was concentrated to half of the volume and diluted with water (500 mL). The resulting mixture was extracted with DiPE (250 mL). The organic phase was washed with 1M NaOH (aq) (300 mL), water (2×300 mL) and brine (150 mL). The solvent was removed under reduced pressure and the oily residue redissolved in DIPE (60 mL) and cooled to -5 °C. The resulting slurry was filtered and the cake was washed with ice cold petrol ether (2×25 mL) to yield **BrFMOL** as pale white crystals (66.55 g, 85% for two steps), mp 75-86 °C; ¹H NMR (CDCl₃) δ 1.60 (s, 6H), 2.02 (s, 1H), 3.87 (s, 3H), 6.62 (d, *J* = 13.2 Hz, 1H), 7.73 (d, *J* = 8.7 Hz, 1H).

The product **BrFMOL** was substantially free of starting material **BrFMAP** (an impurity from which desmethyl impurities are formed) as determined by NMR and HPLC.

### Step 2B: Converting BrFMAP to BrFMOL

A 1 L round bottom flask equipped with an magnetic stirrer, an argon inlet and a temperature probe was charged with the commercially available 3M solution of MeMgCl (100 mL, 0.30 mol) in THF. The solution was cooled to -10 °C, and a solution of acetophenone **BrFMAP** (57.00 g, 0.23 mol) in dry DCM (200 mL) was added dropwise, maintaining the internal temperature below 0 °C. The reaction mixture was then aged at temperature 0 °C for 2 hours and the reaction mixture was quenched first with MeOH (25 mL) and then with 1 M HCl (250 mL), maintaining the internal temperature below 20 °C. The quenched reaction was then aged at 20 °C for 30 min and the layers were cut. The organic phase was washed with water (300 mL) and brine (200 mL). The solvent was removed under reduced pressure to yield **BrFMOL** as a colorless oil (60.10, 99%), which solidifies upon standing.

The product **BrFMOL** was substantially free of desmethyl impurity, preferably free of **BrFMAP** as determined by NMR and HPLC.

.According to the same procedure in various scales, the product, substantially free of desmethyl impurity, is also prepared in toluene and 1,2-dimethoxyethane.

### Step 3: Dehydration of BrFMOL to BrMIPEN

The mixture of **BrFMOL** (40.00 g,152 mmol), polyethylene glycol (PEG400, 40 mL) and 1M H₂SO₄ (120 mL) was refluxed in Dean-Stark apparatus for 12 hours. The pure **BrMIPEN** was collected from the separation part of Dean-Stark as a colorless oil (36.7 g 98%). ¹H NMR (CDCl₃) δ 2.10 (s, 3H), 3.85 (s, 3H), 5.19 (d, 2H), 6.60 (d, *J*= 12.5 Hz, 1H), 7.46 (d, *J* = 8.2 Hz, 1H).

### Example 2: Synthesis of 1-bromo-4-fluoro-5-(2-isopropyl)-2-methoxybenzene (BrMIP)

### Process using Pd/C, 62% HBr:

The solution of **BrMIPEN** (1.58 g, 6.4 mmol) and 62% HBr (0.15 mL) in MeOH (15 mL) was purged with N₂ and 5% Pd-C (0.05 mol%) was added. The mixture was purged with hydrogen and placed under 60 PSI at room temperature until the reaction was complete based on HPLC analysis (15 min). The catalyst was removed by filtration through celite and the solvent was removed under reduced pressure to yield **BrMIP** as a colorless oil (1.56 g, 98%). ¹H NMR (CDCl₃) δ 1.22 (d, *J* = 6.9 Hz, 6H), 3.14 (sept., *J* = 6.9 Hz, 1 H), 3.85 (s, 3H), 6.60 (d, *J* = 11.8 Hz, 1H), 7.37 (d, *J* =8.0 Hz, 1H).

### Process using Pd/C, thiophene:

The solution of **BrMIPEN** (5.10 g, 19.4 mmol) in MeOH (30 mL) was purged with N₂ and thiophene (2.55 mg, 0.05 mol %) was added, followed by of 5% Pd-C (0.05 mol%). The mixture was purged with hydrogen and placed under 60 PSI at room temperature until the reaction was complete based on HPLC analysis. The catalyst was removed by filtration through celite and the solvent was removed under reduced pressure to yield **BrMIP** as a coloriess oil (4.08 g, 85%).

The overall yield for the 4-step process from acetophenone FMAP as described above was up to 82 %. The product was substantially free of desmethyl impurity, preferably free of 1-bromo-5-ethyl-4-fluoro-2-methoxybenzene (**BrMET**) and contains no polysiloxanes as determined by NMR and HPLC.

### Example 3: Synthesis of 4-fluoro-2-methoxy-5-(2-propenyl)phenylboronic acid (MIPENB)

A 500 mL dry flask was charged with 1-bromo-4-fluoro-2-methoxy-5-(2-propenyl)benzene (**BrMIPEN**) (25.05 g, 102 mmol) and dissolved in toluene (80 mL) and THF (80 mL). The resulting solution was flushed with argon, and triisopropyl borate (32 mL, 140 mmol) was added. The mixture was cooled to -80 °C. Then 10 M n-BuLi in hexanes (12.5 mL, 125 mmol) was added slowly, maintaining a temperature below-55°C. Thirty minutes after completion of the n-BuLi addition, the reaction was warmed to -35°C and quenched into 3 M H₂SO₄ solution (75 mL, 0.225 mol). DIPE (200 mL) was added to the mixture to dilute the organic layer. The mixture was stirred (15 min) and the aqueous layer was cut away. The organic layer was washed with 3.0 M H₂SO₄ (75 mL). The organic phase was extracted three times with 1 M NaOH (200 mL first and then 50 mL and 50 mL). The three NaOH extractions were combined, diluted with 2-propanol (85 mL), and cooled to 15 °C. Then the solution was slowly acidified to pH - 2 using 3 M H₂SO₄ (70 mL) while maintaining temperature at 15-20 °C. The resulting slurry was stirred for 1 hour and then filtered. The filter cake was washed with water (3 x 30 mL) and dried under an air flow for 1 day. The filtered solid was placed in an oven under vacuum at 50 °C for 2-3 days to decompose a diaryl impurity and to dry the solid. The off-white crystalline solid was isolated to yield boronic acid **MIPENB** (19.92 g, 93%). ¹H NMR (CDCl₃) δ 2.13 (s, 3H), 3.90 (s, 3H), 5.20 (d, *J* = 8.6 Hz, 2H), 5.91 (s, 2H), 6.62 (d, *J* = 13.0 Hz, 1H), 7.81 (d, *J* = 9.8 Hz, 1H).

### Example 4: Synthesis of 4-fluoro-5-isopropyl-2-methoxyphenylboronic acid (MIPB)

The solution of **MIPENB** (10.05 g, 47.9 mmol) In MeOH (60 mL) was purged with N₂ and 5% Pd-C (0.3 mol%) was added. The mixture was purged with hydrogen and placed under 25 PSI at room temperature until the reaction was complete based on HPLC analysis (30 min). The catalyst was removed by filtration through celite and the solvent was removed under reduced pressure, then water (100 mL) and 2-propanol (20 mL) were added. The mixture was slowly acidified to pH - 2 using 3 M H₂SO₄ while maintaining the temperature below 20 °C. The slurry was stirred for 1h, filtered and the cake was washed with water (2×15 mL). The solid was dried at 50 °C under vacuum to yield **MIPB** as a white solid (9.95 g, 98%). mp 100-102 °C; ¹H NMR (CDCl₃) δ 1.25 (d, *J* = 6.9 Hz, 6H), 3.17 (sept., *J* = 6.9 Hz, 1H), 3.88 (s, 3H), 5.83 (s, 2H), 6.59 (d, *J* =12.4 Hz, 1H), 7.72 (d, *J* = 6.6 Hz, 1 H).

### Example 5: Preparation of biaryis of Formula VII by Suzuki coupling

A 3 M K₂CO₃ solution is prepared by adding solid K₂CO₃ (31 g, 0.22 mol) to water (100 mL). Cooling is applied to keep the solution at 20-25 °C. (2-chloro-5-(trifluoromethyl)phenyl)methanol (Formula **VI',** X = Cl, R₁ = CH₂OH) (17.5 g, 84 mmol), and 4-fluoro-5-isopropyl-2-methoxyphenylboronic acid (**MIPB**) (18.1 g, 85 mmol) are added to the K₂C0₃ followed by all THF (100 mL) rinse. The solution is degassed by sparging with argon gas for 20 min. The catalyst, 1,1-bis(di-tertbutylphosphino)ferrocene palladium dichloride (300 mg, 0.55 mol%) is added. The organic layer turns dark brown immediately. The biphasic mixture is aged at 35-45 °C with vigorous stirring for 32 hours. The mixture is cooled to r. t. and water (150 mL) is added, followed by petrol ether (150 mL) and the aqueous layer is removed. The organic layer was washed with water (2×200 mL) and filtered through silica gel and the solvent is removed under reduced pressure to yield brownish oil which is crystallized from heptane to give 4'-fluoro-5'-isopropyl-2'-methoxy-4-(trifluoromethyl)biphenyl-2-yl)methanol as a pale white solid (28.5 g, 80%). mp 93,5-95,5 °C; ¹H NMR (CDCl₃) δ 1.24 (d, *J* = 6.9 Hz, 6H), 1.95 (t, *J* = 6.1 Hz, 1H), 3.21 (sept., *J* = 6.9 Hz, 1H), 3.73 (s, 3H), 4.49 (m, 2H), 6.68 (d, *J* = 12.0 Hz, 1H), 6.99 (d, *J* = 8.6 Hz, 1H), 7.30 (d, *J* = 7.9 Hz, 1H), 7.59 (dd, *J₁* = 8.0 Hz, *J₂* =1.3 Hz, 1H), 7.86 (d, *J* = 0.7 Hz, 1H).

According to the same procedure in various scales and different concentrations of catalyst some other biaryl of Formula VII₁ and VII₂ are prepared and listed in Table 1 and Table 2, respectively.

**Table 1**

| **VI** (scale) | **VII₁** (R₂) | Pd-cat (mol %) | Crystallization | Yield (%)* | ¹HNMR | Mp (°C) |
|---|---|---|---|---|---|---|
| 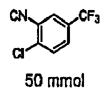 | 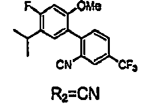 | (A) 0.9 | MeOH/water | 40 | ¹H NMR (CDCl₃) δ 1.27 (d, *J* = 6.9, 6H), 3.22 (sept., *J* = 6.9, 1H), 3.81 (s, 3H), 6.73 (d, *J* = 12.0, 1H), 7.13 (d, *J* = 8.4, 1H), 7.60 (d, *J* = 8.2, 1H), 7.85 (ddd, *J* =8.1, 1.9, 0.5, 1H), 7.97 (s, 1H) | 60-72 |
| 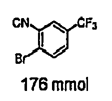 | 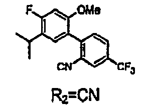 | (A) 0.5 | MeOH/water | 81 | | |
| 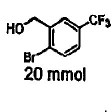 | 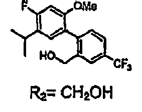 | (A) 0.6 | hexanes | 82 | ¹H NMR(CDCl₃) δ 1.24 (d, *J* = 6.9, 6H), 1.95 (t, *J* = 6.1, 1H), 3.21 (sept., *J* = 6.9, 1H), 3.73 (s, 3H), 4.49 (m, 2H), 6.68 (d, *J* = 12.0, 1H), 6.99 (d, *J* = 8.6, 1H, ArH), 7.30 (d, *J* = 7.9, 1H, ArH), 7.59 (dd, *J* = 8.0, 1.3, 1H, ArH), 7.86 (d, *J* = 0.7, 1H, ArH) | 93-95 |
| 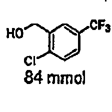 | 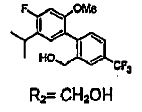 | (A) 0.6 | hexanes | 80 | | |
| 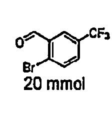 | 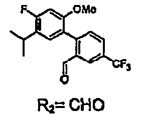 | (B) 1 | MeOH/water | 95 | ¹H NMR (CDCl₃) δ 1.27 (d, *J* = 6.9, 6H), 3.23 (sept., *J* = 6.9, 1H), 3.72 (s, 3H), 6.69 (d, *J* = 11_{.}9, 1H), 7.13 (d, *J* = 8.4, 1H), 7.47 (d, *J* = 8.0, 1H), 7.86 (ddd, *J* = 8.1, 2.0, 0.5, 1H), 8.24 (m, 1H), 9.77 (s, 1H) | 42-48 |
| 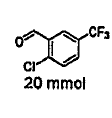 | 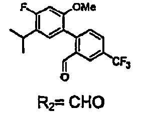 | (B) 1 | MeOH/water | 90 | | |

| | | | | | | |
|---|---|---|---|---|---|---|
| Note: (A): Pd-catalyst was 1,1 bis(di-tertbutylphosphino)ferrocene palladium dichloride (B): Pd-catalyst was Pd(OAc)₂ (1 mol%) with PPh₃ (4 mol%) *Yield after crystallization, turnover of the reactions was higher | | | | | | |

**Table 2**

| **VI (scale)** | **VII₂ (R₂)** | **Pd-cat (mol%)** | **Crystallization** | **Yield (%)*** | **¹HNMR** | **Mp (°C)** |
|---|---|---|---|---|---|---|
| 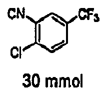 | 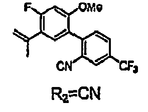 | (A) 0.9 | MeOH/water | 80 | ¹H NMR (CDCl₃) δ 2.15 (m, 3H), 3.84 (s, 3H), 5.21 (m, 1H), 5.26 (m, 1 H), 6.75 (d, *J* = 12.6, 1H), 7.22 (d, *J* = 8.5, 1H), 7.59 (d, *J* = 8.2, 1H), 7.86 (ddd, *J* = 8.3, 1.9, 0.5, 1H). 7.97 (m, 1H) | 89-91 |
| 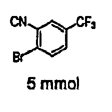 | 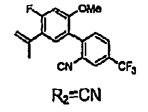 | (A) 0.5 | MeOH/water | 75 | | |
| 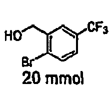 | 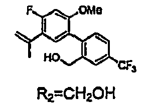 | (A) 0.7 | hexanes | 81 | ¹H NMR (CDCl₃) δ 2.03 (t, *J* = 5.8, 1H), 2.13 (m, 3H), 3.74 (s, 3H), 4.49 (m, 2H), 5.20 (m, 1H), 5.24 (m, 1H), 6.70 (d, *J* = 12.7, 1H), 7.10 (d, *J* = 8.8, 1H), 7.30 (d, *J* = 7.9, 1H), 7.58 (d, *J* = 7.6, 1H, 7.86 (s, 1H) | 113-114 |
| 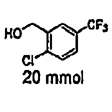 | 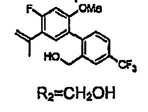 | (A) 0.7 | hexanes | 70 | | |
| 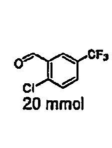 | 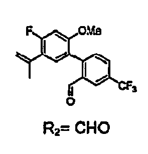 | (B) 1 | hexanes | 87 | ¹H NMR (CDCl₃) δ 2.16 (m, 3H), 3.75 (s, 3H), 5.23 (m, 1H), 5.27 (m, 1H), 6.73 (d, *J* = 15.7, 1H), 7.24 (d, *J* = 10.8, 1H), 7.49 (d, *J* = 10.1, 1H), 7.88 (dd, *J* = 10.0, 2.1, 1H), 8.25 (d, *J* = 1.3, 1H), 9.80 (s, 1H) | 99-102 |
| 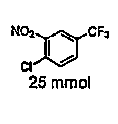 | 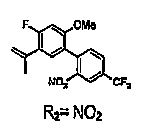 | (B) 1 | Product as an oil | 70 | ¹H NMR (CDCl₃) δ 2.17 (m, 3H), 3.70 (s, 3H), 5.23 (m,1H), 5.28 (m, 1H), 7.26 (d, *J* = 10.7,1H)_{,} 6,64 (d, *J* = 15.7, 1H), 7,56 (d, *J* =10.2, 1N), 7.88 (d, *J* = 10.1.1.5, 1H), 8.20 (d, *J* = 0.9, 1H) | / |

| | | | | | | |
|---|---|---|---|---|---|---|
| Note: (A): Pd-catalyst was 1,1 bis(di-tertbutylphosphino)ferrocene palladium dichloride at room temperature (B): Pd-catalyst was Pd(OAc)₂ (1 mol%) with PPh₃ (4 mol%) at 50 °C *Yield after crystallization, turnover of the reactions was higher | | | | | | |

### Example 6: Preparation of biaryls of Formula VII by Suzuki coupling-one pot process

A 500 mL dry flask was charged with 1-bromo-fluoro-5-isopropyl-2-methoxybenzene (**BrMIP**) (24.60 g, 0.10 mol) and dissolved in toluene (80 mL) and THF (80 mL). The resulting solution was flushed with argon, and tri-isopropylborate (32 mL, 0.14 mol) was added. The mixture was cooled to -80 °C. Then 2.5 M n-BuLi In hexanes (48 mL, 0.12 mol) was added slowly, maintaining a temperature below -55°C. After completion of the n-BuLi addition, the reaction mixture was slowly warmed (1 hour) to -10°C and water (120 mL) was added, followed by commercially available 2-bromo-5-(trifluoromethyl)benzonltrife (Formula **VI'**, X = Br, R₂ = CN) (25.00 g, 0.10 mmol) and the catalyst, 1,1 bis(di-tertbutylphosphino)ferrocene palladium dichloride (265 mg, 0.8 mol%) addition. The organic layer turns dark brown Immediately. The biphasic mixture is aged at room temperature with vigorous stirring for 12 hours. The aqueous layer was removed and the organic layer was washed with 1 M NaOH (aq) (100 mL), water (300 mL) and filtered through silica gel. The solvent was removed under reduced pressure to yield brown oil which was crystallized from EtOH/water (300/100 mL). The resulting slurry was filtered and the filter cake was washed with cold 50% EtOH. The product was dried at 40 °C under vacuum to yield 4'-fluoro-5'-isopropyl-2'-methoxy-4-(trifluoromethyl)biphenyl-2-carbonitrile (Formula **VII'**, R₁ = CN, R₂ = isopropyl) as a pale white solid (25.20 g, 75%).

According to the same procedure in various scales and different concentrations of catalyst some other biaryl of Formula **VII₁** and **VII₂** are prepared and listed in Table 3 and Table 4, respectively. For NMR data and melting points see Table 1 and 2.

**Table 3**

| **VI** (scale) | **VII₁** (R₂) | Pd-cat (mol %) | Crystallization | Yield (%)* |
|---|---|---|---|---|
| 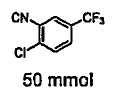 | 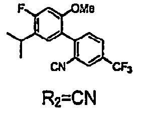 | (A) 0.9 | MeOH/water | 77 |
| 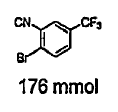 | 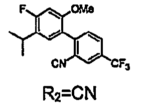 | (A) 0.5 | MeOHlwater | 80 |
| 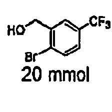 | 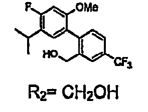 | (A) 0.6 | hexanes | 78 |
| 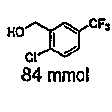 | 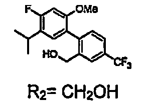 | (A) 0.6 | hexanes | 75 |
| 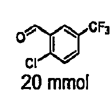 | 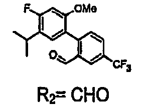 | (B) 1 | MeOH/water | 85 |

| | | | | |
|---|---|---|---|---|
| Note: (A): Pd-catalyst was 1,1 bis(di-tortbutylphosphino)ferrocene palladium dichloride at room temperature (B): Pd-catalyst was Pd(OAc)₂ (1 mol%) with PPh₃(4 mol%) at 50°C *Yield for the two steps and after crystallization, turnover of the reactions was higher | | | | |

**Table 4**

| **VI** (scale) | **VII₂** (R₂) | Pd-cat (mol%) | Crystallization | Yield (%)* |
|---|---|---|---|---|
| 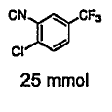 | 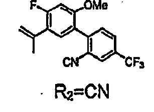 | (A) 0.9 | MeOH/water | 76 |
| 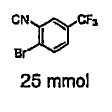 | 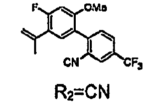 | (A) 0.5 | MeOH/water | 77 |
| 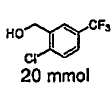 | 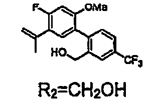 | (A) 0.7 | hexanes | 75 |
| 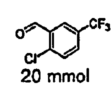 | 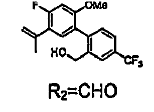 | (A) 0.7 | hexanes | 71 |
| 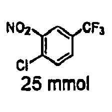 | 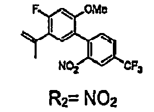 | (B) 1 | Product as an oil | 72 |

| | | | | |
|---|---|---|---|---|
| Note: (A): Pd-catalyst was 1,1 bis(di-tertbutylphosphino)ferrocene palladium dlchloride at room temperature (B): Pd-catalyst was Pd(OAc)₂ (1 mol%) with PPh₃(4 mol%) at 50 °C *Yield for the two steps and after crystallization, turnover of the reactions was higher | | | | |

### Example 7: Synthesis of (4'-fluoro-5'-isopropyl-2'-methoxy-4-(trifluoromethyl)biphenyl-2-yl)methanol (Formula VII₁', R₂ = CH₂OH)

The mixture of (4'-fluoro-2'-methoxy-5'-(prop-1-en-2-yl)-4-(trifluoromethyl)biphenyl-2-yl)methanol (Formula **I₂**, R₁ = CH₂OH) (680 mg, 2 mmol), and 5% Pd/C (25 mg, 0.5 mol%) In MeOH (15 mL) was purged with hydrogen and placed under 30 PSI at room temperature until the reaction was complete based on HPLC analysis. The catalyst was removed by filtration through celite and the solvent was removed under reduced pressure to give 4'-fluoro-5'-isopropyl-2'-methoxy-4-(trifluoromethyl)biphenyl-2-yl)methanol as a pale white solid (671 mg, 98%). mp 93-95 °C; ¹H NMR (CDCl₃) δ 1.24 (d, *J* = 6.9, 6H), 1.95 (t, *J* = 6.1, 1 H), 3.21 (sept., *J* = 6.9, 1H), 3.73 (s, 3H), 4.49 (m, 2H), 6.68 (d, *J* = 12.0, 1H), 6.99 (d, *J* = 8.6, 1H, ArH), 7.30 (d, *J* = 7.9, 1 H, ArH), 7.59 (dd, *J* =8.0,1.3,1 H, ArH), 7.86 (d, *J* = 0.7, 1H, ArH).

### Example 8: Synthesis of (4'-fluoro-5'-isopropyl-2'-methoxy-4-(trifluoromethyl)biphenyl-2-yl)methanamine (Formula VII₁', R₂ = CH₂NH₂)

The mixture of 4'-fluoro-5'-isopropenyl-2'-methoxy-4-(trifluoromethyl)biphenyl-2-carbonitrile (Formula **VII₂'**, R₂ = CN) (3.36 g,10 mmol), 25% NH₄OH (5 mL), and activated Raney Ni (400 mg) in EtOH (40 mL) was purged with hydrogen and placed under 60 PSI at room temperature until the reaction was complete based on HPLC analysis. The catalyst was removed by filtration through celite and the solvent was removed under reduced pressure to give a crude product as a high viscous oil. The oil was crystallized from petrol ether (5 mL) and dried at room temperature under vacuum to yield (4'-fluoro-5'-isopropyl-2'-methoxy-4-(trifiuoromethy)biphenyl-2-yl)methanamine as a pale white solid (3.35 g, 98%). ¹H NMR (CDCl₃) δ 1.24 (d, *J* = 6.9, 6H), 1.33 (bs, 2H), 3.20 (sept., *J* = 6.9, 1 H), 3.68 (d, *J* = 7.5, 2H), 3.72 (s, 3H), 6.67 (d, *J* = 12.1, 1H), 6.97 (d, *J*= 8.6, 1H), 7.27 (d, *J* = 6.9, 1H), 7.53 (d, *J* = 6.8, 1H), 7.76 (s, 1H).

### Example 9: Preparation of anacetrapib

(4'-Huoro-5'-isopropyl-2'-methoxy-4-(trifluoromethyl)biphenyl-2-yl)methanol (Formula **VII₁'**, R₂ = CH₂OH), prepared according to Example 5-7 is further converted to anacetrapib according to step 6 and 7 of Example 1. Analogously (4'-fluoro-5'-(2-propenyl)-2'-methoxy-4-(triftuoromethyl)biphenyl-2-yl)methanol (Formula **VII₂'**, R₂ = CH₂OH), prepared according to Example 5 is converted to (4S,5R)-5-[3,5-bis(trifluoromethyl)phenyl]-3-[[2(4-fluoro-2-methoxy-5-(2-propenyl)phenyl)-5-(trifluoromethy)phenyl]methyl]-4-methyl-1,3-oxazolidin-2-one, which is further converted to anacetrapib according to WO 2006/014413, Example 138.

The Intermediate **BFCI** is substantially free of desmethyl impurities, preferably desmethyl impurity **EBFCI**, and the final product anacetrapib (**IX**) is highly pure in view of desmethyl impurity, preferably in view of impurity **DMAP**, analyzed by HPLC and NMR.

## Claims

1. A process for preparing a compound of formula II 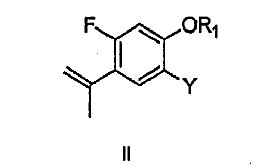
, wherein R₁ is substituted or unsubstituted C₁-C₆ alkyl and Yis Cl, Br or I, by dehydration of a compound of formula I 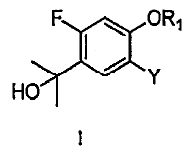 , wherein R₁ and Y are defined as above,
in the presence of a proton donator.

2. The process according to claim 1, **characterized by** either one or a combination of the following features a) to c):
a) in compounds of formula I and II, R₁ is C₁-C₃ alkyl and Y is Br, preferably R₁ is methyl (Me) and Y is Br;
b) water and/or compound of formula II is removed from the reaction mixture during dehydration reaction, preferably water and compound of formula II is removed from the reaction mixture during dehydration reaction, more preferably removal is carried out by distillation, in particular by steam distillation of water and product;
c) the dehydration reaction is optionally carried out in the presence of an organic solvent, miscible with water and inert at acidic conditions, preferably an organic solvent which boiling point is at least 60°C higher than the boiling point of water and/or compound of formula II, more preferably an organic solvent which boiling point is at least 100°C higher than the boiling point of water and/or compound of formula II, even more preferably the organic solvent is non-volatile under applied dehydration reaction conditions, yet even more preferably the organic solvent is polyethylene glycol (PEG), in particular a PEG having an average molecular weight of 200 to 600.

3. A process for preparing a compound of formula III, wherein compound of formula II 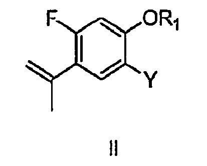 wherein R₁ is substituted or unsubstituted C₁-C₆ alkyl and Y is Cl, Br or I,
is converted to compound of formula III 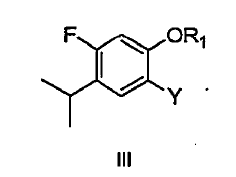 wherein R₁ and Y are defined as above,
by hydrogenation in the presence of a hydrogenation catalyst.

4. The process according to claim 3, **characterized by** either one ore a combination of the following features i) to iv):
i) hydrogenation is carried out in C₁-C₆ alcohol as the solvent.
ii) hydrogenation is carried out using palladium as the hydrogenation catalyst, preferably palladium supported on a solid, more preferably palladium on activated charcoal.
iii) HBr or a catalyst poison is present in the solvent, more preferably the catalyst poison is a sulfur compound, even more preferably the catalyst poison is an organic sulfur component, and in particular the organic sulfur component is thiophen.
iv) conversion of compound of formula II to compound of formula III follows subsequent to a process as defined in claim 1 or 2.

5. The process according to any one of ciaims 1 to 4, wherein compound of formula II or compound of formula III 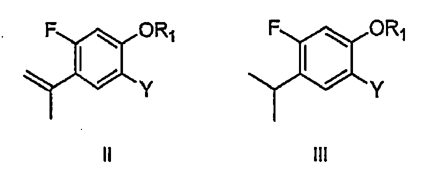 , wherein R₁ substituted or unsubstituted is C₁-C₆ alkyl and Y is Br or I, is respectively converted to compound of formula V or compound of formula IV 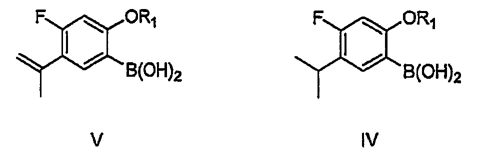 , wherein R₁ is defined as above,
by respectively treating compound of formula II or compound of formula III with trialkyl borate in the presence of an organolithium or lithium In an inert aprotic solvent or mixture of solvents, preferably the trialkyl borate is triisopropyl borate.

6. The process according to any one of claims 1 to 4, wherein compound of formula 11 or compound of formula III 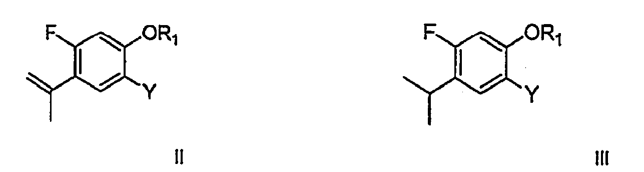 ,wherein R₁ substituted or unsubstituted is C₁-C₆ alkyl and Y is Br or I, is respectively converted to compound of formula VII₂ or compound of formula VII₁ 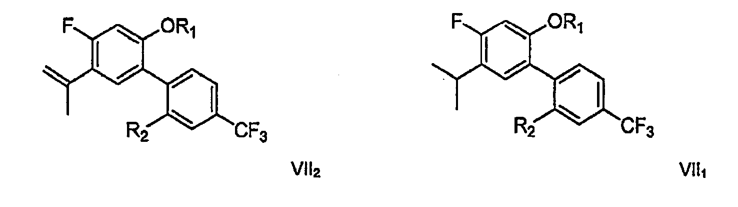 , wherein R₁ is defined as above and wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
by treating compound of formula II or compound of formula III with trialkyl borate in the presence of an organolithium or lithium in an inert aprotic solvent or mixture of solvents followed by an addition of compound of formula VI 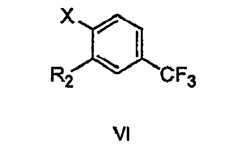 wherein X is Cl, Br, I, -O-SO₂-CF₃ (-OTf), -N₂⁺ or-N(Me)₃⁺, wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro,
in the presence of a coupling catalyst.

7. The process according to claim 6, wherein the synthesis of compound VII₁ or compound VII₂ is carried out in an one-pot reaction.

8. The process according to claims 5 to 7, **characterized by** either one or a combination of the following features (a) to (f):
(a) trialkyl barate is used in an amount of 1.0 to 2.0 equivalents relative to compound of formula II or III and organolithium compound is used in a amount of 1.0 to 1.5 equivalents relative to compound of formula II or III;
(b) trialkyl borate is triisopropyl borate;
(c) organolithium compound is BuLi;
(d) lithium in C₅-C₇-alkane is used;
(e) conversion is carried out at a temperature below -25 °C, preferably below -50 °C;
(f) the catalyst is selected from the group consisting of palladium-triarylphosphine, -trialkylphosphine or - aryl and alklyl substituted phosphine complexes with palladium In the zero oxidation state, salts of palladium in the presence of phosphine ligands, and metallic palladium optionally supported on a solid, preferably the catalyst is Pd(OAc)₂ / PPh₃ or 1,1, bis(di-tertbutylphosphino)ferrocene palladium dichloride

9. The process according to claim 5 or 8, wherein compound of formula V 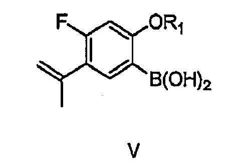 , wherein R₁ is substituted or unsubstituted C₁-C₆ alkyl,
is converted to a compound of formula IV 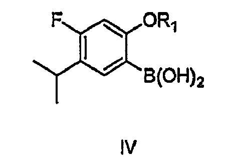 , wherein R₁ is defined as above,
by hydrogenating compound of formula V in the presence of a hydrogenation catalyst, wherein the catalyst is preferably a palladium catalyst wherein hydrogenation is preferably carried out in C₁-C₆ alcohol as the solvent, preferably the palladium catalyst is supported on a solid, more preferably palladium on activated charcoal.

10. A compound having the structural formula 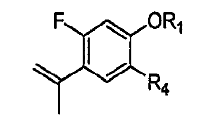 , wherein R₁ is substituted or unsubstituted C₁-C₆ alkyl,
and R₄ is selected from the group consisting of Cl, Br, I, -B(OH)₂ and borate salts and esters, and a moiety having a structural formula 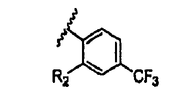 , wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazolyl) and nitro, even more preferably R₂ is cyano, hydroxymethyl, formyl, nitro, aminomethyl.

11. The compound according to claim 10, wherein R₁ is C₁-C₃ alkyl, preferably R₁ is methyl (Me).

12. A process for producing anacetrapib, comprising the steps of:
a) providing compound of formula II 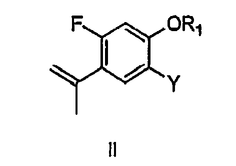 , wherein R₁ is methyl and Y is Cl, Br or I,
by a process according to claim 1 or 2, and
b) subjecting compound of formula II to further synthesis steps to yield anacetrapib.

13. Use of any compound selected from the group consisting of:
a compound of formula II 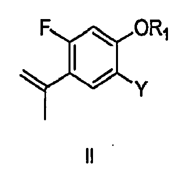 , wherein R₁ is substituted or unsubstituted C₁-C₆ alkyl and Y is Cl, Br or I,
a compound of formula V, 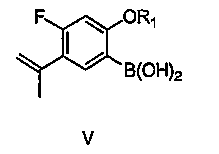 , wherein R₁ is substituted or unsubstituted C₁-C₆ alkyl and Y is Cl, Br or I, and a compound of formula VII₂ 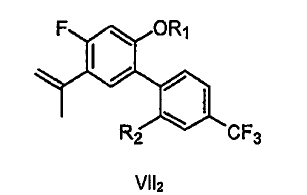
wherein R₂ is CH₂Q or a group which can be converted to CH₂Q group, wherein Q represents a leaving group or a group convertible to a leaving group, preferably Q is selected from the group consisting of halogen, hydroxy, acyl or sulfonyl substituted hydroxy, amino, acyl or sulfonyl substituted amino, more preferably R₂ is selected from the group consisting of hydroxymethyl, alkoxymethyl, halogenmethyl, carboxy, alkoxycarbonyl, amidocarbonyl, cyano, formyl, 2-(4,5-dihydro-1,3-oxazoly) and nitro,
in a process for preparing a pharmaceutically active agent.

14. The use according to claim 13, wherein the pharmaceutically active agent is a compound of formula IX or
any pharmaceutically acceptable salt thereof, 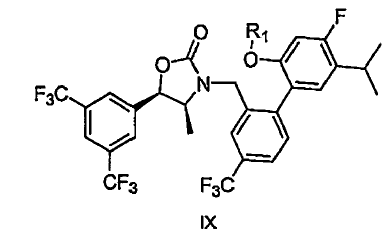 , wherein R₁ is substituted or unsubstituted C₁-C₆ alkyl,
preferably the pharmaceutically active agent is anacetrapib.

15. A process for preparing a pharmaceutical composition comprising anacetrapib as a pharmaceutically active agent, comprising the steps of:
i) preparing anacetrapib according to a process as defined in claim 12, and
ii) admixing the thus prepared anacetrapib with at least one pharmaceutically acceptable exciplent.

16. A pharmaceutical formulation comprising anacetrapib as an active ingredient and at least one pharmaceutically acceptable excipient, wherein anacetrapib is substantially free of desmethylanacetrapib and free of siloxane impurities, preferably anacetrapib is free of both desmethylanacetrapib and siloxane impurities.
